(19) Europäisches Patentamt
European Patent Office
Office européen des brevets

(11) EP 2 477 669 B1

(12) **EUROPÄISCHE PATENTSCHRIFT**

(45) Veröffentlichungstag und Bekanntmachung des Hinweises auf die Patenterteilung:
**22.01.2014 Patentblatt 2014/04**

(21) Anmeldenummer: **11738946.0**

(22) Anmeldetag: **27.05.2011**

(51) Int Cl.:
***A61L 29/08*** *(2006.01)* ***A61L 29/16*** *(2006.01)*

(86) Internationale Anmeldenummer:
**PCT/DE2011/001150**

(87) Internationale Veröffentlichungsnummer:
**WO 2011/147407 (01.12.2011 Gazette 2011/48)**

(54) **KATHETERBALLON BESCHICHTET MIT EINEM ANTIRESTENOTISCHEN WIRKSTOFF UND EINEM TRANSPORTFÖRDERNDEN MOLEKULARDISPERSIONSMITTEL**

BALLOON CATHETER COATED WITH AN ANTI-RESTENOTIC ACTIVE INGREDIENT AND A MOLECULAR DISPERSION AGENT THAT PROMOTES TRANSPORT

BALLONNET DE CATHÉTER REVÊTU D'UN AGENT ANTI-RESTÉNOTIQUE ET D'UN AGENT DE DISPERSION MOLÉCULAIRE FAVORISANT LE TRANSPORT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.05.2010 DE 102010022588**

(43) Veröffentlichungstag der Anmeldung:
**25.07.2012 Patentblatt 2012/30**

(73) Patentinhaber: **Hemoteq AG**
**52146 Würselen (DE)**

(72) Erfinder:
- **HOFFMANN, Erika**
  **52249 Eschweiler (DE)**
- **HOFFMANN, Michael**
  **52249 Eschweiler (DE)**
- **HORRES, Roland**
  **52223 Stolberg (DE)**

(74) Vertreter: **Arth, Hans-Lothar**
**ABK Patent Attorneys**
**Jasminweg 9**
**14052 Berlin (DE)**

(56) Entgegenhaltungen:
**EP-A1- 2 092 942** **WO-A2-2008/086794**
**US-A1- 2010 055 294**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 477 669 B1

## Beschreibung

Die vorliegende Erfindung betrifft kurzzeitig mit dem Organismus in Kontakt kommende medizinische Vorrichtungen wie beispielsweise Ballonkatheter mit oder ohne aufgesetzten Stent, deren Oberfläche die mit mindestens einem antirestenotischen Wirkstoff und einem transportförderndem Molekulardispersionsmittel beschichtet ist, sowie Verfahren zur Herstellung dieser medizinischen Vorrichtungen und deren Verwendung zur Verhinderung oder Verminderung von Restenosen in betroffenen Körperdurchgängen.

Stand der Technik

Nach Einführung von Kurzzeit- als auch Langzeitimplantaten (Stents bzw. Kathetherballons) in Blutgefäße kommt es als Komplikation häufig zu einem Wiederverschluss des Gefäßes, der als Restenose bezeichnet wird. Nach der einschlägigen Fachliteratur kann Restenose als eine Reduktion auf weniger als 50% des Gefäßdurchmessers bezeichnet werden, wobei es sich hier um eine empirische Festlegung handelt.

Die während der zur Behandlung von Stenosen und Verhinderung oder Verminderung von Restenosen implantierten Stent oder verwendeten Katheterballons für Gefäßdehnungen rufen Entzündungsreaktionen hervor, die für den Heilungsprozess in den ersten sieben Tagen eine entscheidende Rolle spielen. Die hierbei ablaufenden Prozesse sind unter anderem mit der Ausschüttung von Wachstumsfaktoren verbunden, womit, eine verstärkte Proliferation der glatten Muskelzellen eingeleitet wird und damit schon kurzfristig zu einer Restenose, einem erneuten Verschluss des Gefäßes aufgrund unkontrollierten Wachstums führen.

Wirkstofffreisetzende Ballonkatheter bieten eine Alternative zum herkömmlichen mit Wirkstoff beschichteten Stent (CardioNews Letter 21.04.2006). Die Patentanmeldung US2010/055294A1 bezieht sich auf Methoden und Vorrichtungen zur Beschichtung von Katheterballons. Die Beschichtungen, z.B.. mit Paclitaxel als antiproliferativem Wirkstoff, sollen besondersgleichmäßig aufgebracht werden. Der Beschichtung können ein Polymer oder ein oberflächenaktiver Stoff als Aditiv hinzugefügt werden. Auch EP2092942A1 offenbart beschichtete Ballonkatheter. Die Beschichtung besteht aus einem Wirkstoff und einem Additive, wobei das Additive ebenfalls ein oberflächenaktiver Stoff sein kann aber auch Fettsäuren oder Fettsäureester.

Das Problem bei den herkömmlichen, mit Wirkstoff und eventuell einer polymerren Matrix beschichteten Katheterballons ist jedoch, den Wirkstoff während des Einführens des Katheterballons ausreichend fest an die Ballonoberfläche zu binden, um ein vorzeitiges Abwaschen im Blutstrom zu verhindern und dennoch bei den Dilatation innerhalb weniger Minuten oder nur einer Minute die ausreichende Abgabe des Wirkstoffs von der Ballonoberfläche auf die Gefäßwand zu gewährleisten, um Restenose wirksam zu verhindern oder zu vermindern zu können (WO2004028582A1). Ein großes Problem bei den Ausführungsformen des Standes der Technik besteht jedoch darin, dass bei einer Dilatationszeit von maximal einer Minute und eventuell mehrerer Wiederholungen der Dilatation nicht genügend antirestenotischer Wirkstoff auf den betroffenen Gefäßabschnitt übertragen werden kann, so dass auch bei einer Dilatation duch einen Katheterballon ohne eingesetzten Stent die Restenose nicht wirksam verhindert wird. Da bei vor allem beim Einsatz in Herzkranzgefäßen durch längere Dilatationszeiten das Herzinfarktrisiko steigt, bleibt insgesamt nur wenig Zeit für die Übertragung des Wirkstoffs oder der Wirkstoffe auf die bzw. die Gefäßwand. Weitere Probleme des Stands der Technik sind geringe Übertragungsmengen des Wirkstoffs oder der Wirkstoffe in die Gefäßwand, keine Kontrolle über die Dosierung, Probleme mit dem Ballonmaterial etc.. Ein weiteres Problem ist das Heranbringen des Wirkstoffs zum Wirkort, da sich während Einführung und Leitung des Katheterballons in der Blutbahn zum Wirkort Teile der Beschichtung ablösen können und dadurch eine unbekannte Menge an Wirkstoff zu der betroffenen Stelle gelangen. Somit ist die Effektivität von solcherart beschichteten Katheterballons zur antirestenotischen Behandlung individuell und unkontrolliert.

Aufgabe der vorliegenden Erfindung ist es, ein Beschichtungssystem bereitzustellen, das das vorzeitige Lösens der Wirkstoffe von der Oberfläche der Katheterballons wirksam verringert und die Abgabe des Wirkstoffs von der Ballonoberfläche auf die Gefäßwand in einer kürzeren Zeitspanne als einer Minute in möglichst hochwirksamer Form gewährleistet.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

## Beschreibung der Erfindung

Überraschend wurde gefunden, dass eine Kombination aus einem antirestenotischen Wirkstoff und einem transportförderndem Molekulardispersionsmittel, welches kein Polymer ist, diese Aufgabe bestmöglichst löst.

Somit betrifft die vorliegende Erfindung einen Katheterballon mit oder ohne aufgesetzen Stent, wobei die Oberfläche des Katheterballons zumindest teilweise mit mindestens einem antirestenotischen Wirkstoff und mindestens

einem transportfördernden Molekulardispersionsmittel gemäß der allgemeinen Formel I beschichtet ist.

Die Erfindung betrifft also Kathetherballons, mit oder ohne aufgesetzen Stent, mit einer Beschichtung aus antirestenotischem Wirkstoff und einem transportförderndem Molekulardispersionsmittel gemäß der allgemeinen Formel I, wobei die Beschichtungskombination eine ausreichende Haft- und Zersetzungsstabilität des Wirkstoffs gewährleistet und eine hohe Freisetzungskinetik aufweist.

Die Erfindung ist vorteilhaft, weil keine oder nur sehr wenig Wirkstoff in der Blutbahn verloren geht und dadurch die Menge des Wirkstoffs am Wirkort genau bestimmt werden kann. Weiterhin wird schon bei kurzer Kontaktzeit des Katheterballons mit der Gefäßwand eine kontrollierbare und optimale Wirkstoffübertragung von der Oberfläche des Katheterballons auf die Gefäßwand gewährleistet. Zudem liegt der Wirkstoff in dem transportförderndem Molekulardispersionsmittel in einer Form vor, welche die Bildung von Partikeln oder Kristallen bzw. von größeren Partikeln oder Kristallen verhindert, weil Partikel und Kristalle und insbesondere größere Partikel und Kristalle keine antirestenotische Wirkung mehr zeigen und nur die applizierte Wirkstoffmenge erhöhen, ohne einen messbaren therapeutischen Effekt zu erzielen. Somit ist fein oder molekular verteilter Wirkstoff in dem transportfördernden Molekulardispersionsmittel bevorzugt, weill dann bei der Dilatation nur aktive Wirkstoffmoleküle an die Gefäßwand abgegeben werden und das transportfördernde Molekulardispersionsmittel zudem für eine gute Übertragung des Wirkstoffs auf die Gefäßwand während der sehr kurzen Dilatationszeit von ca. 1 Minute sorgt.

Die vorliegende Erfindung betrifft somit Katheterballons, welche eine wirkstofffreisetzende Beschichtung aufweisen. Der Begriff Katheterballon oder herkömmlicher Katheterballon wie in der vorliegenden Anmeldung verwendet, bezieht sich auf Katheterballons, Bifurkationsballons, Faltenballons, Angioplastieballons, PTCA-Ballons sowie Spezialballons wie beispielsweise Schlitzballons oder Nadelballons.

Dabei kennzeichnet der Begriff "herkömmliche Katheterballons" dilatierbare Katheterballons, die benutzt werden um mittels Dilatation ein Gefäß vor allem ein Blutgefäß aufzuweiten und optional gleichzeitig einen Stent zu platzieren. Auch nicht dilatierbare Katheterballons für die Stentplatzierung werden unter dem Begriff geführt, welche für selbstexpandierende Stents geeignet sind und zur Verhinderung der vorzeitigen Stentexpansion eine entfernbare Schutzhülle über dem Stent tragen.

Expandierbare und wieder komprimierbare Katheterballons mit einer Schutzhülle wie bei den nicht-dilatierbaren Katheterballons für selbstexpandierende Stents werden jedoch in der Regel ohne Stent eingesetzt, um die auf dem Katheterballon befindliche Beschichtung vor frühzeitiger Ablösung zu schützen.

Bifurkationsballons bezeichnet Katheterballons zur Behandlung einer Verzweigung eines Gefäßes insbesondere eines Blutgefäßes. Derartige Ballons können zwei Arme aufweisen oder aus zwei miteinander verbundenen oder aus zwei getrennten Ballons bestehen, welche gleichzeitig oder nacheinander zur Behandlung einer Gefäßgabelung bzw. zur Platzierung eines Stents oder zweier Stents in einer Gefäßgabelung oder in unmittelbarer Nähe zu einer Gefäßgabelung eingesetzt werden.

Als "Faltenballons" werden Ballons bezeichnet, wie sie beispielsweise in EP 1189553 B1, EP 0519063 B1, WO 03/059430 A1 und WO 94/23787 A1 beschrieben sind und welche "Falten" im komprimierten Zustand des Ballons aufweisen, die sich bei der Expansion des Ballons zumindest teilweise öffnen.

Als Spezialballons werden Ballons mit Poren, insbesondere mit Mikroporen bezeichnet, welche bei der Expansion oder bei dem Anlegen von Druck den Durchtritt von Flüssigkeiten und Lösungen erlauben. Ein solcher Ballon mit Mikroöffnungen ist in EP 0 383 429 A offenbart. Ferner werden unter dem Begriff "Spezialballon" auch Ballons mit speziell ausgestalteter Oberfläche bezeichnet wie beispielsweise der in WO 02/043796 A2 beschriebene Katheterballon mit Mikronadeln oder der in WO 03/026718 A1 offenbart Katheterballon mit einer mirkorauen oder nanorauen Oberfläche zur Einlagerung von Wirkstoffen mit oder ohne Trägersubstanzen.

Der Begriff "Ballon" oder "Katheterballon" bezeichnet grundsätzlich jede expandierbare und wieder komprimierbare sowie temporär implantierbare medizinische Vorrichtung, welche in der Regel zusammen mit einem Katheter verwendet wird.

Die erfindungsgemäßen beschichteten Ballons können ohne Stent als auch mit gekrimptem Stent eingesetzt werden. Ihr Einsatz beschränkt sich hierbei nicht auf eine Erstbehandlung von stenotischem Gefässen, sondern erstreckt sich im Sinne der Erfindung besonders darauf, eine auftretende Re-Stenose (z.B. In-stent-Restenose) erfolgreich zu bekämpfen und eine wiederholte Verengung zu verhindern.

Der Katheterballon kann aus den gängigen Materialien, insbesondere Polymeren bestehen, wie sie weiter unter beschrieben werden und insbesondere aus Polyamid, wie z.B. PA 12, Polyester, Polyurethan, Polyacrylaten, Polyethern usw.

Der Stent kann ebenfalls aus den gängigen Materialien wie beispielsweise medizinischem Edelstahl, Titan, Chrom, Vanadium, Wolfram, Molybdän, Gold, Nitinol, Magnesium, Eisen, Legierungen der vorgenannten Metalle als auch aus polymerem Material und vorzugsweise resorbierbarem polymerem Material wie z.B. Chitosan, Heparanen, Polyhydroxybutyrate (PHB), Polyglyceriden, Polylactiden und Copolymeren der vorgenannten Stoffe.

Vorzugsweise werden die erfindungsgemäßen beschichteten Katheterballons ohne aufgesetzten Stent verwendet, jedoch ist eine Verwendung mit aufgesetztem Stent ebenfalls möglich und bevorzugt. Wird neben dem beschichteten Ballon ein darauf aufgesetztem Stent verwendet, so kann der Stent unbeschichtet (bare stent) oder ebenfalls beschichtet sein, wobei der Stent eine andere Beschichtung und auch einen anderen Wirkstoff als die Beschichtung des Katheterballons aufweisen kann.

Der Begriff "Beschichtung" soll nicht nur eine Beschichtung der Oberfläche des Katheterballons sondern auch eine Befüllung oder Beschichtung von Falten, Kavitäten, Poren, Mikronadeln oder anderen befüllbaren Räumen auf oder zwischen oder in dem Ballonmaterial umfassen.

Unter Wirkstoffen versteht man Substanzen, die eine pharmakologische Aktivität besitzen. Antirestenotische Wirkstoffe umfassen Stoffe, die die Proliferation glatter Muskelzellen hemmen, die andernfalls zum Wiederverschluss eines aufgedehnten Gefäßes führen würde. Bevorzugte äntirestenotische Wirkstoffe gemäß der vorliegenden Erfindung umfassen Paclitaxel, Docetaxel, Rapamycin (Sirolimus), Biolimus A9, Zotarolimus, Everolimus, Myolimus, Novolimus, Pimecrolimus, Täcrolimus, Ridaforolimus und Temsirotimus. Dabei ist die Verwendung von Paclitaxel besonders bevorzugt. Weiterhin bevorzugt ist die Verwendung von Sirolimus oder Sirolimus zusammen mit Paclitaxel.

Grundsätzlich können beliebige Wirkstoffe auch als Wirkstoffkombinationen eingesetzt werden. Bevorzugt sind dabei jedoch Kombinationen von Paclitaxel bzw. Sirolimus mit den anderen Wirkstoffen.

Als Lösungsmittel werden leichtflüchtige organische Verbindungen verwendet wie beispielsweise Dichlormethan, Chloroform, Ethanol, Aceton, Heptan, n-Hexan, DMF, DMSO, Methanol, Propanol, Tetrahydrofuran (THF), Methylenchlorid, Methylacetat, Ethylacetat, Ether, Petrolether, Acetonitril, Essigsäureethyl- und -methylester, Cyclohexan und entsprechende Mischungen. Je nach Beschichtungsmaterial (z:B. Hydrogele oder wasserlösliche Wirkstoffe) kann auch die Anwesenheit von Wasser erwünscht sein. Insbesondere bevorzugt werden Aceton, Ethanol und Ethylacetat. Je nach Wirkstoff und transportförderndem Molekulardispersionsmittel können auch polare Lösungsmittel eingesetzt werden wie beispielsweise Glycerin, Ethylenglykol oder Wasser.

Molekulardispersionsmittel gemäß der allgemeinen Formel I sind in Sinne der Erfindung Stoffe, die mit dem mindestens einen antirestenotischen Wirkstoff ein Gemisch bilden, ohne miteinander chemisch zu reagieren. Ferner soll der antirestenotische Wirkstoff in dem transportfördernden Molekulardispersionsmittel fein verteilt bis zu molekular und in Partikelgrößen < 1 nm vorliegen. Diese erfindungsgemäße Beschichtung ist vorteilhaft, weil sich der oder die Wirkstoffe fein und vorzugsweise homogen verteilt mit dem transportfördernden Molekulardispersionsmittel gemäß der allgemeinen Formel I befinden und nicht in der Blutbahn vorzeitig vom Katheterballon abgelöst werden. Somit ist in einer bevorzugten Ausführungsform der vorliegenden Erfindung der mindestens eine Wirkstoff in dem mindestens einen transportfördernden Molekulardispersionsmittel gemäß der allgemeinen Formel I eingebettet oder eingelagert vorzugsweise in molekularer Form oder als Partikel mit einer durchschnittlichen Partikelgröße kleiner als 1 nm.

Transportfördernd werden Stoffe genannt, die den Transfer des mindestens einen Wirkstoffes von der Oberfläche des Katheterballons zu bzw. in die Zellen und/oder das Gewebe der Gefäßwand im Vergleich zu einem Transfer desselben Wirkstoffs ohne Transportförderer steigern. Transportfördernd sind also Stoffe, die die Aufnahme von Wirkstoffen in die Gefäßwand oder die Übertragung von Wirkstoffen auf die Gefäßwand beschleunigen bzw. erleichtern, so dass der vorhandene Wirkstoff bzw. die Wirkstoffkombination während des kurzzeitigen Kontaktes kontrolliert und in der vorgesehenen Dosierung auf die Gefäßwand übertragen werden kann.

Im Vergleich zum Stand der Technik wird der mindestens eine Wirkstoff innerhalb einer Zeitspanne von wenigen Minuten, bevorzugt einer Minute, noch bevorzugter 50 Sekunden, noch bevorzugter 40 Sekunden und insbesondere bevorzugt 30 Sekunden von der Ballonoberfläche auf die Zellwand übertragen. Das Merkmal, innerhalb einer kurzen Zeitspanne einen maximalen Teil des mindestens einen Wirkstoffs auf die Gefäßwand zu übertragen ist auch deshalb vorteilhaft, weil durch den kurzen Kontakt des Katheterballons mit der Gefäßwand und insbesondere in Herzkranzgefäßen durch den nur kurzzeitigen Verschluss des Gefäßes das Risiko eines Herzinfarkts gesenkt wird. Auch senkt die kurze Dilatationszeit das Risiko von Mikrorissen in den Gefäßen.

Gemäß der Erfindung dienen transportfördernde Molekulardispersionsmittel gemäß der allgemeinen Formel I dazu, den mindestens einen Wirkstoff während der Leitung des Katheterballons auf dessen Öberfläche zu halten und am Wirkort während der Dilatation des Katheterballons den optimalen Transfer des mindestens einen Wirkstoffes zu gewährleisten.

Es ist gemäß der Erfindung besonders bevorzugt, dass das Mengenverhältnis von dem mindestens einen antirestenotischen Wirkstoff und dem mindestens einen transportfördernden Molekulardispersionsmittel gemäß der allgemeinen Formel I von 95 Gew.-% antirestenotischen Wirkstoff zu 5 Gew.-% transportfördernden Molekulardispersionsmittel gemäß der allgemeinen Formel I bis 5 Gew.-% antirestenotischen Wirkstoff zu 95 Gew.-% transportfördernden Molekulardispersionsmittel gemäß der allgemeinen Formel I beträgt. In einer bevorzugten Ausführungsform beträgt das Mengenverhältnis des mindestens einen antirestenotischen Wirkstoffs und dem mindestens einen transportfördernden Molekulardispersionsmittel gemäß der allgemeinen Formel I von 90 Gew.-% antirestenotischen Wirkstoff zu 10 Gew.-% transportfördernden Molekulardispersionsmittel gemäß der allgemeinen Formel I bis 10 Gew.-% antirestenotischen Wirkstoff zu 90 Gew.-% transportfördernden Molekulardispersionsmittel gemäß der allgemeinen Formel I. Noch bevorzugter ist ein Mengenverhältnis von antirestenotischem Wirkstoff und transportförderndem Molekulardispersionsmittel gemäß der allgemeinen Formel 1 von 95 Gew.-% antirestenotischen Wirkstoff zu 5 Gew.-% transportförderndem Molekulardispersionsmittel gemäß der allgemeinen Formel I bis zu 70 Gew.-% antirestenotischen Wirkstoff zu 30 Gew.-% transportförderndem Molekulardispersionsmittel gemäß der allgemeinen Formel I.

Erfindungsgemäß werden als transportfördernde Molekulardispersionsmittel Verbindungen der folgenden allgemeinen Formel (I) verwendet:

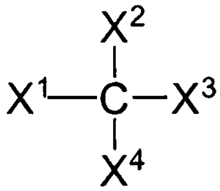

worin

$X^1$ einen der folgenden Reste bedeutet

$-L^1-R^{18}$, $-C(R^3)(R^4)-L^1-R^{18}$, $-L^1-C(R^3)(R^4)-R^{18}$, $-C(R^3)(R^4)-L^1-C(R^5)(R^6)-R^{18}$, $-L^1-Y-R^{18}$, $-Y-L^1-R^{18}$, $-Y-C(R^3)(R^4)-L^1-R^{18}$, $-C(R^3)(R^4)-Y-L^1-R^{18}$, $-C(R^3)(R^4)-L^1-Y-R^{18}$, $-Y-L^1-C(R^3)(R^4)-R^{18}$, $-L^1-Y-C(R^3)(R^4)-R^{18}$, $-L^1-C(R^3)(R^4)-Y-R^{18}$, $-Y-C(R^3)(R^4)-L^1-C(R^5)(R^6)-R^{18}$, $-C(R^3)(R^4)-Y-L^1-C(R^5)(R^6)-R^{18}$, $-C(R^3)(R^4)-L^1-Y-C(R^5)(R^6)-R^{18}$, $-C(R^3)(R^4)-L^1-C(R^5)(R^6)-Y-R^{18}$;

$X^2$ einen der folgenden Reste bedeutet $-R^7$, $(-CH_2-)_p-R^7$, $(-O-CH_2-)_p-R^7$;

$X^3$ einen der folgenden Reste bedeutet
$-M^1-R^{26}$, $-M^1-M^2-R^{26}$, $-M^1-(M^2),M^3-R^{26}$, $-M^1-(M^2),M^3-(M^4)_s-R^{26}$;

$X^4$ einen der folgenden Reste bedeutet
$-L^2-R^{19}$, $-C(R^{10})(R^{11})-L^2-R^{19}$, $-L^2-C(R^{10})(R^{11})-R^{19}$, $-C(R^{10})(R^{11})-C(R^{12})(R^{13})-L^2-R^{19}$, $-C(R^{10})(R^{11})-L^2$ $-C(R^{12})(R^{13})-R^{19}$, $-L^2-C(R^{10})(R^{11})-C(R^{12})(R^{13})-R^{19}$;

$L^1$ für einen der folgenden Gruppen steht

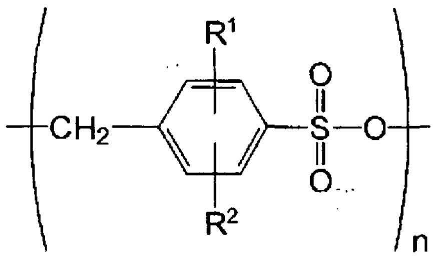

Linker 1

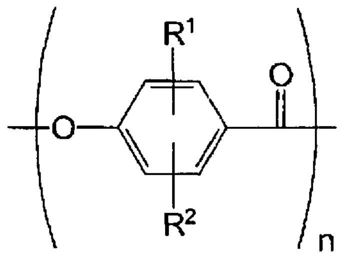

Linker 2

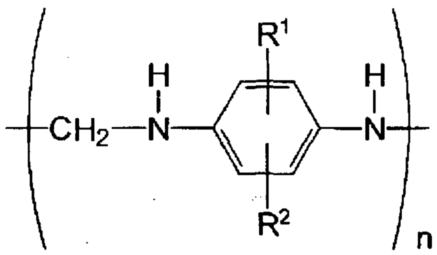

Linker 3

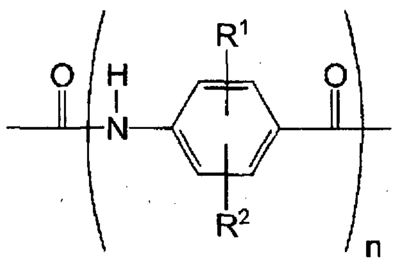

Linker 4

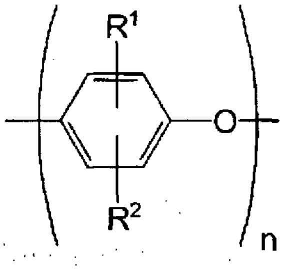

Linker 5

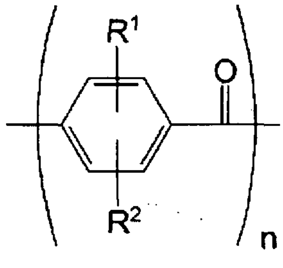

Linker 6

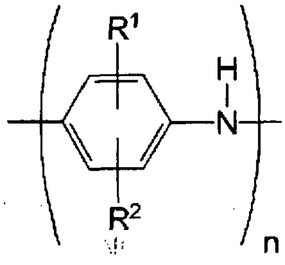

Linker 7

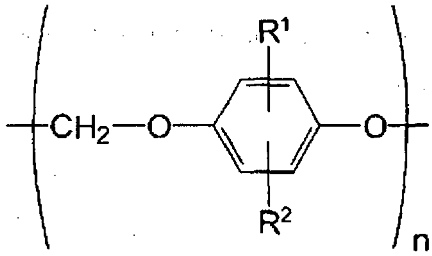

Linker 8

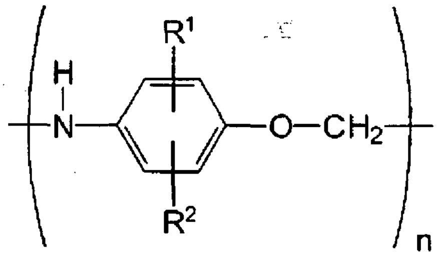

Linker 9

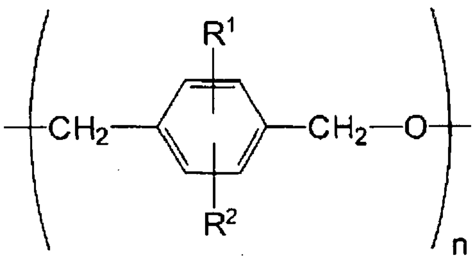

Linker 10

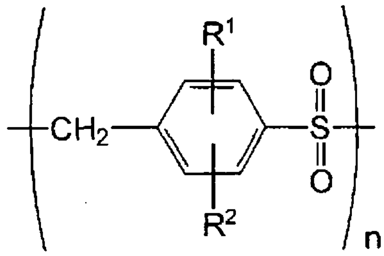

Linker 11

$L^2$ für einen der folgenden Gruppen steht -O-CO-, -NH-CO-, -CO-, -O-, -NH-, -CO-O-, -CO-NH-, -NH-CO-O-, -O-CO-NH-, -O-CO-O-, -NH-CO-NH-;

$M^1$ für einen der folgenden Gruppen steht

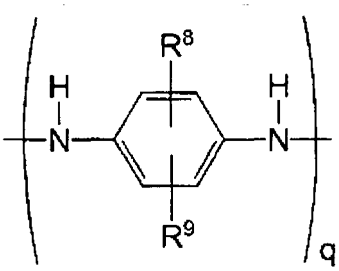

Gruppe 1

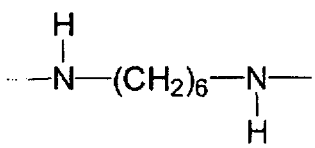

Gruppe 2

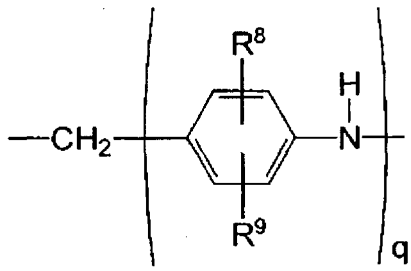

Gruppe 3

$M^2$ für einen der folgenden Gruppen steht -$CH_2$-, -$CH_2$-$CH_2$-, -$CH_2$-$CH_2$-$CH_2$-, -$CH_2$-$CH_2$-$CH_2$-$CH_2$-, -O-, -O-$CH_2$-, -O-CH2-CH2-, -O-$CH_2$-$CH_2$-$CH_2$-, -O-$CH_2$-$CH_2$-$CH_2$-$CH_2$-, -O-CO-, -O-CO-$CH_2$--, -O-CO-$CH_2$-$CH_2$-, -O-CO-$CH_2$-$CH_2$-$CH_2$-, -O-CO-$CH_2$-$CH_2$-$CH_2CH_2$-, -CO-, -CO-$CH_2$-, -CO-$CH_2$-$CH_2$-, -CO-$CH_2$-$CH_2$-$CH_2$-, -CO-$CH_2$-$CH_2$-$CH_2$-$CH_2$-;

$M^3$ für einen der folgenden Gruppen steht eine Bindung, -NH-, -NH-CO-, -NH-CO-NH-, -NH-CS-, -NH-CS-NH-, -NHC(NH)-NH-;

$M^4$ für einen der folgenden Gruppen steht
$(-CH_2-O-CH_2-)_t$, $(-O-CH_2-CH_2-)_t$, $(-CH_2-CH_2-O-)_t$;

Y für $(-CH_2-)_m$, $(-CH_2-O-)_m$, $(-O-CH_2-)_m$, $(-CH_2-CH_2-O-)_m$ oder $(-CH_2-CH_2-CH_2-O-)_m$ steht;

$R^1$ bis $R^{13}$ unabhängig voneinander folgende Reste bedeuten: $-R^{14}$ bis $-R^{30}$, -OH, $-OCH_3$, $-OC_2H_5$, $-OC_3H_7$, -O-cyclo-$C_3H_5$, $-OCH(CH_3)_2$,- $OC(CH_3)_3$. $-OC_4H_9$, -OPh, $-OCH_2$-Ph, $-OCPh_3$, -SH, $-SCH_3$, $-SC_2H_5$, $-SC_3H_7$, -S-cyclo-$C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, -COCN, $-COOCH_3$, $-COOC_2H_5$, $-COOC_3H_7$, -COO-cyclo-$C_3H_5$, $-COOCH(CH_3)_2$, $-COOC(CH_{3)3}$, $-O-CO-R^{14}$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, -NHCO-O-cyclo-$C_3H_5$, $-NHCO-OCH(CH_3)_2$, $-NHCO-OC(CH_3)_3$. $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $-SOCH_3$, $-SOC_2H_5$, $-SOC_3H_7$, -SO-cyclo-$C_3H_5$, $-SOCH(CH_3)_2$, $-SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$, $-SO_3$-cyclo-$C_3H_5$, $-SO_3CH(CH_3)_2$, $-SO_3C(CH_3)_3$, $-SO_2NH_2$, $-OCF_3$, $-OC_2F_5$, $-O-COOCH_3$, $-O-COOC_2H_5$, $-O-COOC_3H_7$, -O-COO-cyclo-$C_3H_5$, $-O-COOCH(CH_3)_2$, $-O-COOC(CH_3)_3$, $-NH-CO-NH_2$, $-NH-CO-NHCH_3$, $-NH-CO-NHC_2H_5$, $-NH-CO-NHC_3H_7$, -NH-CO-NH-cyclo-$C_3H_5$, $-NH-CO-NH[CH(CH_3)_2]$, $-NH-CO-NH[C(CH_3)_3]$, $-NH-CO-N(CH_3)_2$, $-NH-CO-N(C_2H_5)_2$, $-NH-CO-N(C_3H_7)_2$, -NH-CO-N(cyclo-$C_3H_5)_2$, $-NH-CO-N[CH(CH_3)_2]_2$, $-NH-CO-N[C(CH_3)_3]_2$, $-NH-CS-NH_2$, $-NH-CS-NHCH_3$, $-NH=CS-NHC_2H_5$, $-NH-CS-NHC_3H_7$, -NH-CS-NH-cyclo-$C_3H_5$, $-NH-CS-NH[CH(CH_3)_2]$, $-NH-CS-NH[C(CH_3)_3]$, $-NH-CS-N(CH_3)_2$, $-NH-CS-N(C_2H_5)_2$, $-NH-CS-N(C_3H_7)_2$, -NH-CS-N(cyclo-$C_3H_5)_2$, $-NH-CS-[N[CH(CH_3)_2]_2$, $-NH-CS-N[C(CH_3)_3]_2$, $-NH-C(=NH)-NH_2$, $-NH-C(=NH)-NHCH_3$, $-NH-C(=NH)-NHC_2H_5$, $-NH-C(=NH)-NHC_3H_7$, -NH-C(=NH)-NH-cyclo-$C_3H_5$, $-NH-C(=NH)-NH[CH(CH_3)_2]$, $-NH-C(=NH)-NH[C(CH_3)_3]$, $-NH-C(=NH)-N(CH_3)_2$, $-NH-C(=NH)-N(C_2H_5)_2$, $-NH-C(=NH)-N(C_3H_7)_2$, -NH-C(=NH)-N(cyclo-$C_3H_5)_2$, $-NH-C(=NH)-N[CH(CH_3)_2]_2$, $-NH-C(=NH)-N[C(CH_3)_3]_2$, $-O-CO-NH_2$, $-O-CO-NHCH_3$, $-O-CO-NHC_2H_5$, $-O-CO-NHC_3H_7$, -O-CO-NH-cyclo-$C_3H_5$, $-O-CO-NH[CH(CH_3)_2]$, $-O-CO-NH[C(CH_3)_3]$, $-O-CO-N(CH_3)_2$, $-O-CO-N(C_2H_5)_2$, $-O-CO-N(C_3H_7)_2$, -O-CO-N(cyclo-$C_3H_5)_2$, $-O-CO-N[CH(CH_3)_2]_2$,- $O-CO-N[C(CH_3)_3]_2$, $-O-CO-OCH_3$, $-O-CO-OC_2H_5$, $-O-CO-OC_3H_7$, -O-CO-O-cyclo-$C_3H_5$, $-O-CO-OCH(CH_3)_2$, $-O-CO-OC(CH_3)_3$;

$R^{14}$ bis $R^{30}$ unabhängig voneinander folgende Reste bedeuten:

- $CH_2F$, $-CHF_2$, $-CF_3$, $-CH_2Cl$, $-CH_2Br$, $-CH_2I$, $-CH_2-CH_2F$, $-CH_2-CHF_2$,$-CH_2-CF_3$, $-CH_2-CH_2Cl$, $-CH_2-CH_2Br$, $-CH_2-CH_2I$, cyclo-$C_3H_5$, cyclo-$C_4H_7$, cyclo-$C_5H_9$, cyclo-$C_6H_{11}$, cyclo-$C_7H_{13}$, cyclo-$C_8H_{15}$, -Ph, $-CH_2$-Ph, $-CPh_3$, -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$. $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$. $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3\}=CH_2$, $-CH(CH_3)-CH=CH$, $-CH=C(CH_3)_2$, $-(CH_3)=CH-CH_3$, $-CH=CH_.CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$, $-CH=CH-CH=CH-CH_3$, $-CH=CH-CH_2-CH=CH_2$, $--C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$. $-CH_2-CH=C(CH_3)_{2,}$ $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-_{C(CH3)}c_{CH}-_{CH}=_{CH2}$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6--C(CH_3)=CH_2$, $-C_2H_4-CH(CH_3)-CH=CH_2$, $-CH_2-CH(CH_3)-CH_2-CH=CH_2$. $-CH(CH_3)-C_2H_4-CH=CH_2$, $-C_2H_4-CH=C(CH_3)_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$. $-CH_2-CH=C(CH_3)-C_2H_5$. $-CH_2-C(CH_3)=CH-C_2H_5$, $-CH(CH_3)-CH=CH-C_2H_{5,}$ $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-CH(CH_3)-CH_2-C(CH-_3)=CH_2$, $-CH(CH_3)-CH(CH_3)-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)_=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)$

$=C(CH_3)_2$, $-C[C(CH_3)_3]=CH_2$,- $C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-CH=CH-C_2H_4-CH=CH_2$, $-CH_2-CH=CH-CH=CH-CH_3$, $-CH=CH-CH_2-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH=CH-CH=CH_2$, $-CH=CH-CH_2-C(CH_3)=CH_2$, $-CH=CH-CH(CH_3)-CH=CH_2$, $-CH=C(CH_3)-CH_2-CH=CH_2$, $-C(CH_3)=CH-CH_2-CH=CH_2$, $-CH=CH-CH=C(CH_3)_2$, $-CH=CH-C(CH_3)=CH-CH_3$, $-CH=C(CH_3)-CH=CH-CH_3$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_8-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-CH_2-CH(CH_3)-C\equiv CH$, $-CH(CH_3)-CH_2-C\equiv CH$, $-CH(CH_3)-C\equiv C-CH_3$, $-C_4H_8-C\equiv CH$, $-C_3H_6-C\equiv C-CH_3$, $-C_2H_4-C\equiv C-C_2H_5$, $-CH_2-C\equiv C-C_3H_7$, $-C\equiv C-C_4H_9$, $-C_2H_4-CH(CH_3)-C\equiv CH$, $-CH_2-CH(CH_3)-CH_2C\equiv CH$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$. $-CH_2-C\equiv C-CH(CH_3)_2$, $-C\equiv C-CH(CH_3)C_2H_5$, $-C\equiv C-CH_2-CH(CH_3)_2$, $-C\equiv C-C(CH_3)_3$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-C\equiv C-C\equiv CH$, $-CH_2-C\equiv C-C\equiv CH$, $-C\equiv C-C\equiv C-CH_3$, $-CH(C\equiv CH)_2$, $-C_2H_4-C\equiv C-C\equiv CH$, $-CH_2-C\equiv C-CH_2-C\equiv CH$, $-C\equiv C-C_2H_4-C\equiv CH$, $-CH_2-C\equiv C-C\equiv C-CH_3$, $-C\equiv C-CH_2-C\equiv C-CH_3$, $-C=C-C\equiv C-C_2H_5$, $-C\equiv C-CH(CH_3)-C\equiv CH$, $-CH(CH_3)-C\equiv C-C\equiv CH$, $-CH(C\equiv CH)-CH_2-C=CH$, $-C(C\equiv CH)_2-CH_3$, $-CH_2-CH(C\equiv CH)_2$, $-CH(C\equiv CH)-C=C-CH_3$;

m ist eine ganze Zahl von 1 bis 10;

n ist eine ganze Zahl von 0 bis 5;

p ist eine ganze Zahl von 0 bis 3;

q ist eine ganze Zahl von 0 bis 4;

r steht für 0 oder 1;

s steht für 0 oder 1;

t ist eine ganze Zahl von 1 bis 10.

Das transportfördernde Molekulardispersionsmittel der allgemeinen Formel I für die Einbettung des mindestens einen antirestenotischen Wirkstoffs ist dabei durch konkrete physikalische Eigenschaften definiert.

Bei den transportfördernden Molekulardispersionsmitteln gemäß der allgemeinen Formel I handelt es sich um organische Verbindungen, welche Kohlenstoff, Wasserstoff und Sauerstoff und optional Stickstoff enthalten. Enthalten die transportfördernden Molekulardispersionsmittel Stickstoff, so ist der Stickstoff vorzugsweise in Amidbindungen oder als Amingruppe anwesend. Derartige Verbindungen enthalten vorzugsweise 1 bis 5 Amidbindungen, weiter bevorzugt 2 bis 3 Amindbindungen und/oder besitzen 1 bis 5 Amingruppen, wobei die Gesamtzahl der Stickstoffatome zwischen 1 und 10, bevorzugt zwischen 2 und 7, weiter bevorzugt zwischen 3 und 5 liegt. Die stickstoffhaltigen transportfördernden Molekulardispersionsmittel besitzen Schmelzpunkte oberhalb von -20°C, vorzugsweise oberhalb von 0°C, weiter bevorzugt oberhalb von 20°C und insbesondere bevorzugt oberhalb von 40°C und Siedepunkte von mindestens 470°C, bevorzugt von mindestens 490°C, noch bevorzugter von mindestens 510°C, noch bevorzugter von mindestens 530°C, noch bevorzugter von mindestens 550°C, noch bevorzugter von mindestens 570°C, noch bevorzugter von mindestens 590°C, noch bevorzugter von mindestens 610°C und insbesondere bevorzugt einen Siedepunkt von mindestens 630°C. Zudem sollte der Siedepunkt nicht über 650°C liegen. Die Siedepunkte gelten für den Normaldruck auf Meereshöhe, also 760 mm Hg. Eine bevorzugte Subgruppe sind transportfördernde Molekulardispersionsmittel gemäß der allgemeinen Formel I ohne Stickstoff, welche aus Kohlenstoff, Wasserstoff und Sauerstoff bestehen und nicht als Salz vorliegen und auch nicht geladen vorliegen und kein Säureproton tragen. Solche transportfördernden Molekulardispersionsmittel gemäß der allgemeinen Formel (I) weisen bevorzugt einen Siedepunkt von mindestens 400°C, noch bevorzugter von mindestens 420°C, noch bevorzugter von mindestens 440°C, noch bevorzugter von mindestens 460°C, noch bevorzugter von mindestens 470°C, noch bevorzugter von mindestens 480°C, noch bevorzugter von mindestens 490°C und insbesondere bevorzugt einen Siedepunkt von mindestens 500°C auf. Zudem sollte der Siedepunkt nicht über 550°C liegen. Die Siedepunkte gelten für den Normaldruck (1.013 hPa) auf Meereshöhe, also 760 mm Hg. Die transportfördernden Molekulardispersionsmittel enthalten 1 bis 15 Sauerstoffatome, bevorzugter 2 bis 14, noch bevorzugter 3 bis 13, noch bevorzugter 4 bis 12, noch bevorzugter 5 bis 11, noch bevorzugter 6 bis 10, und insbesondere bevorzugt 7 bis 9 Sauerstoffatome. Die Sauerstoffatome sind vorzugsweise in Esterbindungen oder in Etherbindungen enthalten. Des weiteren ist bevorzugt, dass bei der Anwesenheit von Etherbindungen zwischen 4 und 12, bevorzugt zwischen 5 und

11, weiter bevorzugt zwischen 6 und 10 Etherbindungen oder bei der Anwesenheit von Esterbindungen zwischen 2 und 6, bevorzugt zwischen 3 und 5 Esterbindungen vorhanden sind. Die stickstofffreien transportfördernden Molekulardispersionsmittel gemäß der allgemeinen Formel (I) müssen eine molare Masse (Molekulargewicht) von mindestens 400 g/mol, bevorzugter von mindestens 420 g/mol, noch bevorzugter von mindestens 440 g/mol, noch bevorzugter von mindestens 460 g/mol, noch bevorzugter von mindestens 470 g/mol, noch bevorzugter von mindestens 480 g/mol, noch bevorzugter von mindestens 490 g/mol und insbesondere bevorzugt ein Molekulargewicht von mindestens 500 g/mol besitzen. Die stickstoffhaltigen transportfördernden Molekulardispersionsmittel gemäß der allgemeinen Formel (I) müssen eine molare Masse (Molekulargewicht) von mindestens 450 g/mol, bevorzugter von mindestens 460 g/mol, noch bevorzugter von mindestens 470 g/mol, noch bevorzugter von mindestens 480 g/mol, noch bevorzugter von mindestens 490 g/mol, noch bevorzugter von mindestens 500 g/mol, noch bevorzugter von mindestens 510 g/mol und insbesondere bevorzugt ein Molekulargewicht von mindestens 520 g/mol besitzen. Zudem weisen die transportfördernden Molekulardispersionsmittel gemäß der allgemeinen Formel I mit mindestens einer Aminogruppe oder Amidgruppe zwischen 25 und 50 Kohlenstoffatome, bevorzugt zwischen 27 und 48 Kohlenstoffatome, weiter bevorzugt zwischen 29 und 46 Kohlenstoffatome, weiter bevorzugt zwischen 31 und 44 Kohlenstoffatome und insbesondere bevorzugt zwischen 33 und 42 Kohlenstoffatome auf, wobei die stickstofffreien transportfördernden Molekulardispersionsmittel zwischen 20 und 40 Kohlenstoffatome, bevorzugt zwischen 22 und 38 Kohlenstoffatome, weiter bevorzugt zwischen 24 und 36 Kohlenstoffatome, weiter bevorzugt zwischen 26 und 34 Kohlenstoffatome und insbesondere bevorzugt zwischen 28 und 32 Kohlenstoffatome aufweisen. Eine bevorzugte Gruppe der transportfördernden Molekulardispersionsmittel ohne Stickstoff besitzt vorzugsweise einen Schmelzpunkt oberhalb von -80°C, noch bevorzugter oberhalb von -75°C, noch bevorzugter oberhalb von -70°C, noch bevorzugter oberhalb von -65°C und insbesondere bevorzugt einen Schmelzpunkt oberhalb von -60°C. Diese Schmelzpunkte werden insbesondere durch lineare Alkylreste erhalten. Ferner hat sich herausgestellt, dass die transportfördernden Molekulardispersionsmittel der allgemeinen Formel (I) weiterhin eine Dichte von 0,80 g/cm$^3$ bis 1,20 g/cm$^3$, noch bevorzugter von 0,85 g/cm$^3$ bis 1,15 g/cm$^3$, noch bevorzugter von 0,90 g/cm$^3$ bis 1,10 g/cm$^3$, noch bevorzugter von 0,93 g/cm$^3$ bis 1,12 g/cm$^3$, und insbesondere bevorzugt eine Dichte von 0,95 g/cm$^3$ bis 1,05 g/cm$^3$ haben sollten. Des weiteren wurde gefunden, dass die bevorzugten transportfördernden Molekulardispersionsmittel der allgemeinen Formel (I) einen Flammpunkt oberhalb von 50°C, bevorzugter oberhalb von 60°C, noch bevorzugter oberhalb von 70°C, noch bevorzugter oberhalb von 80°C, noch bevorzugter oberhalb von 85°C, weiterhin noch bevorzugter oberhalb von 90°C, noch bevorzugter oberhalb von 95°C und insbesondere bevorzugt einen Flammpunkt oberhalb von 100°C besitzen. Die gemessenen Brechungsindices der bevorzugten transportfördernden Molekulardispersionsmittel gemäß allgemeiner Formel (I) lagen über 1,40 und unter 1,50, so dass ein Brechungsindex $n_D^{20}$ zwischen 1,400 und 1,500 bevorzugt ist und weiter bevorzugt ein Brechungsindex $n_D^{20}$ zwischen 1,410 und 1,490, noch bevorzugter wischen 1,420 und 1,480, noch bevorzugter wischen 1,430 und 1,470, noch bevorzugter wischen 1,435 und 1,465 und insbesondere bevorzugt ist ein Brechungsindex $n_D^{20}$ zwischen 1,440 und 1,460. Es sei angemerkt, dass solche transportfördernden Molekulardispersionsmittel der allgemeinen Formel I umso bevorzugter sind, umso mehr die oben erwähnten chemischen und physikalischen Parameter in den bevorzugten Bereichen liegen. Am besten geeignet sind daher solche transportfördernden Molekulardispersionsmittel gemäß der allgemeinen Formel I, welche alle vorgenannten Parameter in den am meisten bevorzugten Bereichen haben. Ein stickstoffhaltiges transportförderndes Molekulardispersionsmittel mit einem Brechungsindex $n_D^{20}$ von 1,49 und einem Siedepunkt von 480°C und einer Dichte von 1,18 g/cm$^3$ ist somit weniger bevorzugt als ein stickstofffreies transportförderndes Molekulardispersions-mittel mit einem Brechungsindex $n_D^{20}$ von 1,44 und einem Siedepunkt von 510°C und einer Dichte von 1,00 g/cm$^3$, sofern alle anderen Parameter beider Verbindungen außerhalb der bevorzugten Bereiche liegen. Zudem sei erwähnt, dass die Bestimmung des Brechungsindex, des Siedepunktes, des Schmelzpunktes, des Flammpunktes als auch der Dichte mittels dem Fachmann wohl bekannter Standardmethoden erfolgt ist.

Die in Tabelle 1 beschriebenen 19 transportfördernden Molekulardispersionsmittel wurden als Beschichtungsmaterialien für Katheterballons zusammen mit einem antirestenotischen Wirkstoff getestet. Die 19 Verbindungen sind entweder kommerziell erhältlich oder durch einfache Synthesen und Standardreaktionen wie z.B. Veresterung oder Herstellung von Amidbindungen zugänglich.

**Tabelle 1**

| Reste / Verbindung | $X^1$ | $X^2$ | $X^3$ | $X^4$ |
|---|---|---|---|---|
| Verb.1 | $-Y-L^1-R^{18}$<br>Y = $-CH_2-$ | $-COO-C_2H_5$ | $-M^1-(M^2)-M^3-R^{26}$<br>$M^1$=Gruppe 1 (q=0) | $-C(R^{10})(R^{11})-L^2-R^{19}$ |

(fortgesetzt)

| Reste / Verbindung | $X^1$ | $X^2$ | $X^3$ | $X^4$ |
|---|---|---|---|---|
| | $L^1$ = Linker 5, (n=1)<br>$R^1 = R^2$ = -H<br>$R^{18}$ = cyclo-$C_3H_5$ | | $M^2$=-$CH_2$-, (r = 0)<br>$M^3$= -NH-<br>$R^{26}$ = cyclo-$C_3H_5$ | $L^2$ = -NH-CO-<br>$R^{10} = R^{11}$ = -H<br>$R^{19}$ = -$CH_3$ |
| Verb.2 | -Y-$L^1$-C($R^3$)($R^4$)-$R^{18}$<br>Y =-$C_2H_4$-<br>$L^1$ = Linker 2, (n=1)<br>$R^1 = R^2$ = -H<br>$R^3 = R^4$ = -H<br>$R^{18}$ = -$C_5H_{11}$ | -$OC_3H_7$ | -$M^1$-($M^2$)$_r$-$M^3$-($M^4$)$_s$-$R^{26}$<br>$M^1$=Gruppe 2 (q=0)<br>$M^2$= -O-, (r = 1);<br>$M^3$= Bindung<br>$M^4$= (-$C_2H_4O$-)$_5$, (s=1); $R^{26}$ = -$C_2H_5$ | -C($R^{10}$)($R^{11}$)-$L^2$-C($R^{12}$)($R^{13}$)-$R^{19}$<br>$L^2$ = -O-CO-<br>$R^{10} = R^{11}$ = -H<br>= $R^{13}$ =-H<br>$R^{19}$ = -$C_4H_9$ |
| Verb.3 | -$L^1$-C($R^3$)($R^4$)-Y-$R^{18}$ | -$OCH_2$- | -$M^1$-$R^{26}$ | -$L^2$-C($R^{10}$) |
| | $L^1$ = Linker 3, (n=1)<br>$R^1 = R^2$ = -H<br>$R^3 = R^4$ = -H<br>$R^{18}$ = -$CH_3$ | | $R^8 = R^9$ = -H<br>$R^{26}$ = -$C_2H_5$ | $L^2$ = -NH-CO-<br>$R^{10} = R^{11}$= -H<br>$R^{19}$ = -$CH_2$-$CF_3$ |
| Verb.4 | -C($R^3$)($R^4$)-$L^1$-Y-C($R^5$)($R^6$)-$R^{18}$<br>Y = -$CH_2$-<br>$L^1$ = Linker 4, (n=1)<br>$R^1 = R^2$= -H<br>$R^3 = R^4$= -H<br>$R^5 = R^6$ = -H<br>R1$^8$ = -C≡C-$CH_3$ | -$CONH_2$ | -$M^1$-($M^2$)$_r$-$M^3$-($M^4$)$_s$-$R^{26}$<br>$M^1$=Gruppe 3 (q=0)<br>$M^2$= -O-, (r = 1)<br>$M^3$= -NH-CO-<br>$M^4$= -$OC_2H_4$-, (s=0)<br>$R^{26}$ = -$C_4H_9$ | -$L^2$-C($R^{10}$)($R^{11}$) -C($R^{12}$)($R^{13}$)-$R^{19}$<br>$L^2$ = -CO-NH-<br>$R^{10} = R^{11}$ = -H<br>$R^{12} = R^{13}$ = -H<br>$R^{19}$= -$C_6H_{13}$ |
| Verb.5 | -$L^1$-Y-C($R^3$)($R^4$)-$R^{18}$<br>Y = -$CH_2$-<br>$L^1$ = Linker 5, (n=1)<br>$R^1 = R^2$ = -H<br>$R^3 = R^4$ = -H<br>$R^{18}$ = -$CH_3$ | -COOcyclo-$C_3H_5$ | -$M^1$-($M^2$)$_r$$M^3$-($M^4$)$_s$-$R^{26}$<br>$M^1$=Gruppe 2 (q=0)<br>$M^2$= -$CH_2$-, (r = 1);<br>$M^3$= -NH-CO-<br>$M^4$ = -$C_2H_4O$-, (s=1); $R^{26}$ = -$CH_3$ | -C($R^{10}$)($R^{11}$)-C($R^{12}$)($R^{13}$)-$L^2$-$R^{19}$<br>$L^2$ = -COO-<br>$R^{10} = R^{11}$ = -H<br>$R^{12} = R^{13}$ = -H<br>$R^{19}$ = -$C_4H_9$ |
| Verb.6 | -C($R^3$)($R^4$)-$L^1$-$R^{18}$<br>$L^1$ = Linker 1, (n=2)<br>$R^1 = R^2$ = -H<br>$R^3 = R^4$ = -H<br>$R^{18}$ = -$CH_3$ | -CO-$OCH_3$ | -$M^1$-$R^{26}$<br>$M^1$=Gruppe 1 (q=0)<br>$R^{26}$ = -$C_4H_9$ | -C($R^{10}$)($R^{11}$)-$L^2$-$R^{19}$<br>$L^2$ = -CO-<br>$R^{10} = R^{11}$ = -H<br>$R^{19}$ = -$C_6H_{13}$ |
| Verb.7 | -$L^1$-Y-$R^{18}$<br>Y = (-$CH_2CH_2O$-)$_m$<br>m = 3<br>$L^1$ = Linker 6, (n=1)<br>$R^1 = R^2$ = -H<br>$R^{18}$ = -$C_2H_5$ | -$OCH_2$-$OCH_3$ | -$M^1$-$R^{26}$<br>$M^1$=Gruppe 1 (q=0)<br>$R^{26}$ = -$C_6H_{13}$ | -$L^2$-C($R^{10}$) ($R^{11}$)-$R^{19}$<br>$L^2$ = -O-<br>$R^{10} = R^{11}$ = -H<br>$R^{19}$ = -$C_4H_9$ |

(fortgesetzt)

| Reste / Verbindung | $X^1$ | $X^2$ | $X^3$ | $X^4$ |
|---|---|---|---|---|
| Verb.8 | $-C(R^3)(R^4)-L^1-C(R^5)(R^6)-R^{18}$<br>$L^1$ = Linker 4, (n=1)<br>$R^1 = R^2$ -H<br>$R^3 = R^4$ = -H<br>$R^5 = R^6$ -H<br>$R^{18} = -C_2H_5$ | $-N(Me)_2$<br>Me= $CH_3$ | $-M^1-(M^2)_rM^3-(M^4)_s-R^{26}$<br>$M^1$=Gruppe 2 (q=0)<br>$M^2$= $-COCH_2CH_2-$, (r = 1);<br>$M^3$ = Bindung<br>$M^4$= $(-OC_2H_4-)_4$, (s=1);<br>$R^{26}$ = -H | $-C(R^{10})(R^{11})-L^2-C(R^{12})(R^{13})-R^{19}$<br>$L^2$ = -O-CO-<br>$R^{10} = R^{11}$ = -H<br>$R^{12} = R^{13}$ = -H<br>$R^{19} = -CH_3$ |
| Verb.9 | $-L^1-C(R^3)(R^4)-R^{18}$ | -COOH | $-M^1-(M^2)_r-M^3-R^{26}$ | $-L^2-R^{19}$ |
| | $L^1$ = Linker 7, (n=1)<br>$R^1 = R^2$ = -H<br>$R^3 = R^4$ = -H<br>$R^{18} = -C_6H_{13}$ | | $M^1$=Gruppe 1 (q=0)<br>$M^2$= $-CH_2-$, (r = 0)<br>$M^3$= -NH-CO-<br>$R^{26} = -C_2H_5$ | $L^2$ = -N H-CO-O-<br>$R^{19}$ = cyclo-$C_3H_5$ |
| Verb.10 | $-Y-C(R^3)(R^4)-L^1-R^{18}$<br>Y = $(-CH_2O-)_m$<br>m = 4<br>$L^1$ = Linker 8, (n=1)<br>$R^1 = R^2$ = -H<br>$R^3 = R^4$ = -H<br>$R^{18} = -CH_3$ | $-CH_2-OC_2H_5$ | $-M^1-(M^2)-M^3-(M^4)_s-R^{26}$<br>$M^1$=Gruppe 2 (q=0)<br>$M^2$= $-CH_2-$, (r = 0);<br>$M^3$= -NH-CO-<br>$M^4$=$-C_2H_4O-$, (s=1);<br>$R^{26}$ = cyclo-$C_5H_9$ | $-C(R^{10})(R^{11})-L^2-R^{19}$<br>$L^2$ = -CO-NH-<br>$R^{10} = R^{11}$ = -H<br>$R^{19} = -C_5H_{11}$ |
| Verb.11 | $-C(R^3)(R^4)-Y-L^1-R^{18}$<br>Y = $(-OCH_2-)_m$<br>m = 2<br>$L^1$ = Linker 5, (n=1)<br>$R^1 = R^2$ = -H<br>$R^3 = R^4$ = -H<br>$R^{18} = -CH_3$ | $-OCH_2-OH$ | $-M^1-(M^2)_r-M^3-(M^4)_s-R^{26}$<br>$M^1$=Gruppe 3 (q=0)<br>$M^2$=$-COCH_2-$, (r=1)<br>$M^3$= -NH-<br>$M^4$= $-OC_2H_4-$, (s=0)<br>$R^{26} = -C_4H_9$ | $-C(R^{10})(R^{11})-C(R^{12})(R^{13})-L^2-R^{19}$<br>$L^2$ = -CO-<br>$R^{10} = R^{11}$ =-H<br>$R^{12} = R^{13}$ = -H<br>$R^{19}$=$-CH_2-C{\equiv}CH$ |
| Verb.12 | $-C(R^3)(R^4)-L^1-Y-R^{18}$<br>Y = $(-CH_2CH_2O-)_m$<br>m = 4<br>$L^1$ = Linker 4, (n=0)<br>$R^1 = R^2$ = -H<br>$R^3 = R^4$ = -H<br>$R^{18} = -CH_3$ | $-O-C(CH_3)_3$ | $-M^1-(M^2)_r-M^3-(M^4)_s-R^{26}$<br>$M^1$=Gruppe 1 (q=0)<br>$M^2$= -O-CO-$CH_2-CH_2-CH_2-$, (r = 1)<br>$M^3$= Bindung<br>$M^4$= $-C_2H_4O-$, (s=1)<br>$R^{26} = -C_3H_7$ | $-L^2-C(R^{10})(R^{11})-C(R^{12})(R^{13})-R^{19}$<br>$L^2$ = -O-CO-<br>$R^{10} = R^{11}$ = -H<br>$R^{12} = R^{13}$ =-H<br>$R^{19} = -C_5H_{11}$ |
| Verb.13 | $-L^1-R^{18}$<br>$L^1$ = Linker 2, (n=3)<br>$R^1 = R^2$= -H<br>$R^{18} = -C_4H_9$ | $-O-CO-C_3H_7$ | $-M^1-R^{26}$<br>$M^1$=Gruppe 1 (q=0)<br>$R^{26} = -C_5H_{11}$ | $-L^2-R^{19}$<br>$L^2$ = -O-CO-O-<br>$R^{19} = -C_3H_7$ |
| Verb.14 | $-Y-C(R^3)(R^4)-L^1-C(R^5)(R^6)-R^{18}$<br>Y = $-CH_2O-$<br>$L^1$ = Linker11, (n=1)<br>$R^1 = R^2$ = -H<br>$R^3 = R^4$ = -H<br>$R^5 = R^6$ = -H | $-CH_2$ $OC_2H_5$ | $-M^1-M^2-R^{26}$ $M^1$=Gruppe 2 (q=1)<br>$M^2$= $-CH_2-CH_2-$<br>$R^{26} = -C_5H_{11}$ | $-C(R^{10})(R^{11})-L^2-C(R^{12})(R^{13})-R^{19}$<br>$L^2$ = -CO-<br>$R^{10} = R^{11}$ = -H<br>$R^{12} = R^{13}$ = -H<br>$R^{19} = -C_6H_{13}$ |

(fortgesetzt)

| Reste / Verbindung | $X^1$ | $X^2$ | $X^3$ | $X^4$ |
|---|---|---|---|---|
| | $R^{18}$ = $-OC_2H_5$ | | | |
| Verb.15 | $-C(R^3)(R^4)-Y-L^1-C(R^5)(R^6)-R^{18}$<br>Y = $-OCH_2-$<br>$L^1$ = Linker 2, (n=1)<br>$R^1 = R^2 = -H$<br>$R^3 = R^4 = -H$<br>$R^5 = R^6 = -H$<br>$R^{18} = -CH(CH_3)_2$ | -O-cyclo-$C_3H_5$ | $-M^1-(M^2)r-M^3-R^{26}$<br>$M^1$=Gruppe 1 (q=1)<br>$M^2$= $-CH_2-$, (r = 1)<br>$M^3$= -NH-CO-<br>$R^8 = R^9 = -H$<br>$R^{26} = -CH_3$ | $-L^2-C(R^{10})(R^{11})-R^{19}$<br><br>$L^2$ = -O-CO-<br>$R^{10} = R^{11} = -H$<br>$R^{19} = -H$ |
| Verb.16 | $-C(R^3)(R^4)-L^1-C(R^5)(R^6)-Y-R^{18}$<br>Y = $-CH_2-$<br>$L^1$ = Linker10, (n=1)<br>$R^1 = R^2 = -H$<br>$R^3 = R^4 = -H$<br>$R^5 = R^6 = -H$<br>$R^{18} = -H$ | $-O-CH(Me)_2$<br><br><br>Me= $CH_3$ | $-M^1-(M^2)_r-M^3-(M^4)_s-R^{26}$<br><br>$M^1$ = Gruppe 1 (q=0)<br>$M^2$= $-CH_2-$, (r = 0)<br>$M^3$= -NH-CO-<br>$M^4$= $-OC_2H_4-$, (s=0)<br>$R^{26} = -C_2H_5$ | $-L_2-C(R^{10})(R^{11})-C(R^{12})(R^{13})-R^{19}$<br><br>$L^2$ = -COO-<br>$R^{10} = R^{11} = -H$<br>$R^{12} = R^{13} = -H$<br>$R^{19} = -C_4H_9$ |
| Verb.17 | $-L^1-Y-R^{18}$<br>Y = $-CH_2O-$<br>$L^1$ = Linker 5, (n=1)<br>$R^1 = R^2 = -H$<br>$R^{18} = -C_3H_7$ | $-O-CO-OC_2H_5$ | $-M^1-(M^2)_r-M^3-R^{26}$<br>$M^1$=Gruppe 2 (q=1)<br>$M^2$= $-CH_2CH_2CH_2-$, (r=1)<br><br>$M^3$= -NH-$R^{26}$ = $-C_2H_5$ | $-L^2-C(R^{10})(R^{11})-R^{19}$<br><br>$L^2$ = -CO-<br>$R^{10} = R^{11} = -H$<br>$R^{19} = -CH(CH_3)_2$ |
| Verb.18 | $-Y-L^1-R^{18}$<br>Y = $-CH_2-CH_2-$<br>$L^1$ = Linker 9, (n=1)<br>$R^1 = R^2 = -H$<br>$R^{18}$ = -cyclo-$C_3H_5$ | $-OC_2H_5$ | $-M^1-(M^2)_r-M^3-(M^4)_s-R^{26}$<br>$M^1$=Gruppe 2 (q=0)<br>$M^2$= $-O-CO-CH_2-$, (r=1);<br>$M^3$= -NH-<br>$M^4$= $-OC_2H_4-$, (s=0)<br>$R^{26} = -C_6H_{13}$ | $-C(R^{10})(R^{11})-C(R^{12})(R^{13})-L^2-R^{19}$<br><br>$L^2$ = -CO-NH-<br>$R^{10} = R^{11} = -H$<br>$R^{12} = R^{13} = -H$<br>$R^{19} = -C_2H_5$ |
| Verb.19 | $-L^1-Y-C(R^3)(R^4)-R^{18}$<br>Y = $-CH_2-$<br>$L^1$ = Linker 7, (n=1)<br>$R^1 = R^2 = -H$<br>$R^3 = R^4 = -H$<br>$R^{18} = -H$ | $-CO-OC_3H_7$ - | $-M^1-M^2-R^{26}$<br><br>$M^1$=Gruppe 3 (q=1)<br>$M^2$= -CO-<br>$R^8 = R^9 = -H$<br>$R^{26} = -C_3H_7$ | $-L^2-R^{19}$ $L^2$ = -O-CO-NH-<br><br><br>$R^{19} = -C_6H_{13}$ |

Für die vollständig oder partielle Beschichtung eines Katheterballons mit oder ohne aufgesetzten Stent wird eine Lösung bestehend aus dem mindestens einen antirestenotischen Wirkstoff und dem mindestens einem transportfördernden Molekulardispersionsmittels und dem Lösungsmittel oder Lösungsmittelgemisch inklusive eventueller Zusatzstoffe durch Sprüh-, Tauch-, Pinsel-, Spritzen-, Schlepp-, Roll- oder Pipettierverfahren oder Elektrospinning auf die Katheterballonoberfläche aufgetragen. Der Katheterballon lässt sich entweder im expandierten oder im gefalteten Zustand beschichten, teilweise oder vollständig beschichten oder zusammen mit einem aufgesetzten Stent beschichten. Diese Beschichtungsverfahren sind Stand der Technik und in der Offenlegungsschrift der internationalen Patentanmeldung WO2008086794 ausführlich offenbart. Als optionale Zusatzstoffe können Phosphatidylinositol, Phosphatidylserin, Phosphatidylcholin, Phosphatidylethanolamin, Phosphatidsäure oder andere Phosphatidylverbindungen in Mengen bis zu 50 Gew.-% bezogen auf alle Komponenten der Beschichtung eingesetzt werden.

Der Begriff "Beschichtung" soll nicht nur eine Beschichtung der Oberfläche des Katheterballons sondern auch eine Befüllung oder Beschichtung von Falten, Kavitäten, Poren, Mikronadeln oder anderen befüllbaren Räumen auf

oder zwischen oder in dem Ballonmaterial umfassen.

Die Trockung der erfindungsgemäßen Beschichtung auf der Ballonoberfläche kann z.B. durch Stehenlassen an der Luft (Verdunstung des Lösungsmittels) oder durch Erwärmung und/oder verminderten Druck (Vakuum) bzw. Rotationstrocknung erfolgen, die in Offenlegungsschrift der internationalen Patentanmeldung WO2008086794 ausführlich beschrieben ist. Dadurch wird das Lösungsmittel entfernt, so dass die Beschichtung aus dem mindestens einen Wirkstoff und dem mindestens einen transportfördernden Molekulardispersionsmittel besteht.

Die Beschichtungslösung enthält also mindestens einen Wirkstoff, mindestens ein transportförderndes Molekulardispersionsmittef gemäß der allgemeinen Formel I, und mindestens ein Lösungsmittel.

Die folgenden Beispiele beschreiben die vorliegende Erfindung ohne diese auf die konkreten Ausführungsformen zu beschränken.

**Beispiele**

**Beispiel 1**: Beschichtung eines Katheterballons mit Paclitaxel und Verbindung 1

Ein nichtexpandierter Katheterballon wird rotierbar auf eine horizontale Stange gelagert. Eine Lösung von 90 Gew.-% Paclitaxel in Ethanol und 10 Gew.-% der Verbindung 1 wird mit Hilfe einer Sprühanlage auf die Katheterballonoberfläche aufgebracht. Anschließend wird der Katheterballon langsam drehend (20 Umdrehungen pro Minute) bei Raumtemperatur über Nacht getrocknet.

**Beispiel 2**: Beschichtung eines Katheterballons mit Paclitaxel und Verbindung 2

Ein Katheterballon wird rotierbar auf eine horizontale Stange gelagert und mittels Pipettierverfahren mit einer Lösung aus 85 Gew.-% Pacliltaxel in Methanol und 15 Gew.-% Verbindung 2 beschichtet. Anschließend wird der Katheterballon langsam drehend bei Raumtemperatur mehrere Stunden getrocknet.

**Beispiel 3**: Beschichtung eines Katheterballons mit Paclitaxel und Verbindung 3

Ein Katheterballon wird in eine Lösung aus 80 Gew.-% Pacliltaxel in Aceton und 20 Gew.-% Verbindung 3 getaucht und anschließend unter langsamer Drehung um seine Längsachse bei Raumtemperatur getrocknet. Der Tauchvorgang wird weitere 2 Mal wiederholt.

**Beispiel 4**: Beschichtung eines Katheterballons mit Paclitaxel und Verbindung 4

Ein Katheterballon wird rotierbar auf eine horizontale Stange gelagert und mittels Pipettierverfahren mit einer Lösung aus 90 Gew.-% Pacliltaxel in Ethanol und 10 Gew.-% Verbindung 5 beschichtet. Anschließend wird der Katheterballon langsam drehend bei Raumtemperatur getrocknet.

**Beispiel 5**: Beschichtung eines Katheterballons mit Paclitaxel und Verbindung 5

Ein Katheterballon wird rotierbar auf eine horizontale Stange gelagert und mittels Pipettierverfahren mit einer Lösung aus 83 Ges.-% Pacliltaxel in Ethanol und 17 Gew.-% Verbindung 5 beschichtet. Anschließend wird der Katheterballon langsam drehend bei Raumtemperatur getrocknet.

**Beispiel 6**: Beschichtung eines Katheterballons mit Paclitaxel und Verbindung 6

Ein Katheterballon wird rotierbar auf eine horizontale Stange gelagert und mittels Sprühverfahren mit einer Lösung aus 95 Gew.-% Pacliltaxel in Aceton und 5 Gew.-% Verbindung 6 beschichtet. Anschließend wird der Katheterballon langsam drehend bei Raumtemperatur getrocknet.

**Beispiel 7**: Beschichtung eines Katheterballons mit Paclitaxel und Verbindung 7

Ein Faltenballon wird in deflatiertem Zustand rotierbar auf eine horizontale Stange gelagert und die Falten des Ballons mittels Pipettierverfahren mit einer Lösung aus 90 Gew.-% Pacliltaxel in Ethanol und 10 Gew.-% Verbindung 7 beschichtet. Anschließend wird der Ballon langsam drehend bei Raumtemperatur unter Anlegen eines Vakuums getrocknet.

**Beispiel 8**: Beschichtung eines Katheterballons mit Paclitaxel und Verbindung 8

Ein Katheterballon wird rotierbar auf eine horizontale Stange gelagert und mittels Sprühverfahren mit einer Lösung aus 90 Gew.-% Pacliltaxel in Aceton und 10 Gew.-% des transportfördernden Molekulardispersionsmittels entsprechend Verbindung 8 beschichtet. Anschließend wird der Katheterballon langsam drehend bei Raumtemperatur getrocknet.

**Beispiel 9**: Beschichtung eines Katheterballons mit Paclitaxel und Verbindung 9

Ein Katheterballon wird rotierbar auf eine horizontale Stange gelagert und mittels Sprühverfahren mit einer Lösung aus 86 Gew.-% Pacliltaxel in Aceton und 14 Gew.-% Verbindung 9 vollflächig beschichtet. Anschließend wird der Katheterballon langsam drehend bei Raumtemperatur unter Anlegen eines Vakuums getrocknet.

**Beispiel 10**: Beschichtung eines Katheterballons mit Paclitaxel und Verbindung 10

Ein Katheterballon wird rotierbar auf eine horizontale Stange gelagert und mittels Schleppverfahren mit einer Lösung aus 60 Gew.-% Pacliltaxel in Aceton und 40 Gew.-% Verbindung 10 vollflächig beschichtet. Anschließend wird der Katheterballon langsam drehend bei Raumtemperatur getrocknet.

**Beispiel 11**: Beschichtung eines Katheterballons mit Paclitaxel und Verbindung 11

Ein Katheterballon wird in eine Lösung aus 40 Gew.-% Pacliltaxel in DMSO und 60 Gew.-% Verbindung 11 getaucht. Anschließend wird der Katheterballon langsam drehend bei Raumtemperatur unter Anlegen eines Vakuums getrocknet.

**Beispiel 12**: Beschichtung eines Katheterballons mit Paclitaxel und Verbindung 12

Ein Katheterballon wird rotierbar auf eine horizontale Stange gelagert und mittels Schleppverfahren mit einer Lösung aus 80 Gew.-% Pacliltaxel in Aceton und 20 Gew.-% Verbindung 12 vollflächig bestrichen. Anschließend wird der Katheterballon langsam drehend bei Raumtemperatur getrocknet.

**Beispiel 13**: Beschichtung eines Katheterballons mit Paclitaxel und Verbindung 13

Ein Katheterballon wird rotierbar auf eine horizontale Stange gelagert und mittels Sprühverfahren mit einer Lösung aus 90 Gew.-% Pacliltaxel in Aceton und 10 Gew.-% Verbindung 13 vollflächig beschichtet. Anschließend wird der Katheterballon langsam drehend bei Raumtemperatur getrocknet.

**Beispiel 14**: Beschichtung eines Katheterballons mit Paclitaxel und Verbindung 14

Ein Katheterballon wird rotierbar auf eine horizontale Stange gelagert und mittels Pipettierverfahren mit einer Lösung aus 91 Gew.-% Pacliltaxel in Aceton und 9 Gew.-% Verbindung 14 vollflächig beschichtet. Anschließend wird der Katheterballon langsam drehend bei Raumtemperatur getrocknet.

**Beispiel 15**: Beschichtung eines Katheterballons mit Paclitaxel und Verbindung 15

Ein Katheterballon wird rotierbar auf eine horizontale Stange gelagert und mittels Streichverfahren mit einer Lösung aus 89 Gew.-% Pacliltaxel in Aceton und 11 Gew.-% Verbindung 15 vollflächig beschichtet. Anschließend wird der Katheterballon langsam drehend bei Raumtemperatur getrocknet.

**Beispiel 16**: Beschichtung eines Katheterballons mit Paclitaxel und Verbindung 16

Ein Katheterballon wird rotierbar auf eine horizontale Stange gelagert und mittels Sprühverfahren mit einer Lösung aus 90 Gew.-% Pacliltaxel in Aceton und 10 Gew.-% Verbindung 16 vollflächig beschichtet. Anschließend wird der Katheterballon langsam drehend bei Raumtemperatur getrocknet.

**Beispiel 17**: Beschichtung eines Katheterballons mit Paclitaxel und Verbindung 17

Ein Katheterballon wird rotierbar auf eine horizontale Stange gelagert und mittels Streichverfahren mit einer

Lösung aus 90 Gew.-% Pacliltaxel in Aceton und 10 Gew.-% Verbindung 17 vollflächig beschichtet. Der Beschichtungsvorgang wird zweimal wiederholt. Anschließend wird der Katheterballon langsam drehend bei Raumtemperatur getrocknet.

**Beispiel 18**: Beschichtung eines Katheterballons mit Paclitaxel und Verbindung 18

Ein Katheterballon wird rotierbar auf eine horizontale Stange gelagert und mittels Sprühverfahren mit einer Lösung aus 90 Gew.-% Pacliltaxel in Aceton und 10 Gew.-% Verbindung 18 vollflächig beschichtet. Anschließend wird der Katheterballon langsam drehend bei Raumtemperatur getrocknet.

**Beispiel 19**: Beschichtung eines Katheterballons mit Paclitaxel und Verbindung 19

Ein Katheterballon wird rotierbar auf eine horizontale Stange gelagert und mittels Pinselverfahren mit einer Lösung aus 90 Gew.-% Pacliltaxel in Aceton und 10 Gew.-% Verbindung 19 vollflächig beschichtet. Anschließend wird der Katheterballon langsam drehend bei Raumtemperatur getrocknet.

**Beispiel 20**: Beschichtung eines Katheterballons mit Rapamycin und Verbindung 1

Ein Katheterballon wird rotierbar auf eine horizontale Stange gelagert und mittels Sprühverfahren mit einer Lösung aus 90 Gew.-% Rapamycin in Ethylacetat und 10 Gew.-% Verbindung 1 vollflächig beschichtet. Anschließend wird der Katheterballon langsam drehend bei Raumtemperatur getrocknet.

**Beispiel 21**: Beschichtung eines Katheterballons mit Rapamycin und einem Gemisch aus Verbindung 2 und 3

Ein Katheterballon wird in eine Lösung aus 90 Gew.-% Rapamycin in Ethylacetat und 5 Gew.-% des transportfördernden Molekulardispersionsmittels entsprechend Verbindung 2 und 5 Gew.-% Verbindung 3 vollflächig beschichtet. Anschließend wird der Katheterballon langsam drehend bei Raumtemperatur unter Anlegen eines Vakuums getrocknet.

**Beispiel 22**: Beschichtung eines Katheterballons mit einem Gemisch aus Paclitaxel, Rapamycin und Verbindung 4

Ein Katheterballon wird in eine Lösung aus 45 Gew.-% Paclitaxel, 45 Gew.-% Rapamycin in Ethylacetat und 10 Gew.-% Verbindung 4 vollflächig beschichtet. Anschließend wird der Katheterballon langsam drehend bei Raumtemperatur unter Anlegen eines Vakuums getrocknet.

**Beispiel 23**: Beschichtung eines Katheterballons mit einem aufgesetzten Stent mit einem Paclitaxel und Verbindung 5

Ein Katheterballon wird rotierbar auf eine horizontale Stange gelagert und mittels Sprühverfahren mit einer Lösung aus 90 Gew.-% Pacliltaxel in Ethanol und 10 Gew.-% Verbindung 5 beschichtet. Anschließend wird der Katheterballon langsam drehend bei Raumtemperatur getrocknet. Auf diesen beschichteten Katheterballon wird nun ein Stent aus Polylactid aufgesetzt, der ebenfalls mit der Lösung aus 90 Gew.-% Pacliltaxel in Ethanol und 10 Gew.-% Verbindung 5 beschichtet wird.

**Beispiel 24**: Einsatz eines Katheterballons in stenotischen Gefäßen von Schweinen

Die Effektivität von Katheterballons mit einer Beschichtung aus 90 Gew.-% Paclitaxel und 10 Gew.-% der Verbindungen 1 - 19 wurde im Tierexperiment an Schweinen in überdehnten Koronararterien getestet.

Bis zu drei Koronargefäße (Ramus circumflexus, Ramus interventricularis anterior, rechte Koronararterie) pro Schwein wurden aufgedehnt. Pro Untersuchungsgruppe wurden 10 Koronargefäße behandelt und die Ergebnisse über alle Gefäße gemittelt. Die Ergebnisse sind in Tabelle 2 zusammengefasst. Der Begriff "late lumen loss" bezieht sich auf den Unterschied zwischen den Durchmessern eines Gefäßabschnittes nach der Aufdehnung und dem nach 28 Tagen angefertigten Nachfolgeangiogramm. Zusätzlich wurde der Erfolg des Einsatzes in histopathologischen Untersuchungen analysiert.

**Tabelle 2**

| | Überdehnungsrate (%) | Stenosegrad 28 d (%) | late lumen loss (mm) |
|---|---|---|---|
| unbeschichtet | ca. 10 | 40,4±12,5 | 1,24±0,35 |
| Verbindung 1 + Ptx | ca. 10 | 14,8±2,8 | 0,19±0,15 |

(fortgesetzt)

| | Überdehnungsrate (%) | Stenosegrad 28 d (%) | late lumen loss (mm) |
|---|---|---|---|
| Verbindung 2 + Ptx | ca. 10 | 13,3±1,7 | 0,18±0,13 |
| Verbindung 3 + Ptx | ca. 10 | 15,8±2,5 | 0,19±0,14 |
| Verbindung 4 + Ptx | ca. 10 | 14.7±3,6 | 0,17±0,12 |
| Verbindung 5 + Ptx | ca. 10 | 16,3±3,2 | 0,22±0,15 |
| Verbindung 6 + Ptx | ca. 10 | 15,6±3,8 | 0,20±0,12 |
| Verbindung 7 + Ptx | ca. 10 | 13,8±3,3 | 0,22±0,11 |
| Verbindung 8 + Ptx | ca. 10 | 15,8±2,1 | 0,16±0,15 |
| Verbindung 9 + Ptx | ca. 10 | 14,8±2,3 | 0,21±0,11 |
| Verbindung 10 + Ptx | ca. 10 | 13,4±3,2 | 0,21±0,15 |
| Verbindung 11 + Ptx | ca. 10 | 12,1±1,9 | 0,19±0,10 |
| Verbindung 12 + Ptx | ca. 10 | 17,4±3,2 | 0,17±0,14 |
| Verbindung 13 + Ptx | ca. 10 | 15,3±4,1 | 0,18±0,15 |
| Verbindung 14 + Ptx | ca. 10 | 16,3±3,7 | 0,22±0,12 |
| Verbindung 15 + Ptx | ca. 10 | 10,2±3,4 | 0,21±0,13 |
| Verbindung 16 + Ptx | ca. 10 | 14,8±5,3 | 0,21±0,14 |
| Verbindung 17 + Ptx | ca. 10 | 16,8±3,8 | 0,22±0,12 |
| Verbindung 18 + Ptx | ca. 10 | 15,5±3,2 | 0,18±0,12 |
| Verbindung 19 + Ptx | ca. 10 | 14,9±2,5 | 0,17±0,10 |

Die Restenosedaten der Experimente mit Katheterballons, die mit den Verbindungen 1 - 19 beschichtet waren, zeigten nach 28 Tagen deutlich geringere Werte als die des Katheterballons ohne eine erfindungsgemäße Beschichtung. Die erfindungsgemäße Beschichtung verstärkte die Aufnahme von Paclitaxel in die Gefäßwände deutlich und verringerte die Restenoserate im Vergleich zur Kontrolle signifikant.

<u>**Beispiel 25**: Herstellern einer Stammlösung des Wirkstoffs mit dem transportfördernden Molekulardispersionsmittels</u>

Wirkstoff und transportförderndes Molekulardispersionsmittel werden im Verhältnis 90 Gew.-% zu 10 Gew.-% in Aceton gelöst und die Lösung unter Rühren gemischt.

## Patentansprüche

**1.** Katheterballon mit oder ohne aufgesetzen Stent, wobei die Oberfläche des Katheterballons zumindest teilweise mit mindestens einem antirestenotischen Wirkstoff und mindestens einem transportfördernden Molekulardispersionsmittel beschichtet ist, wobei das mindestens eine transportfördernde Molekulardispersionsmittel mindestens eine Verbindung der allgemeinen Formel (I) ist:

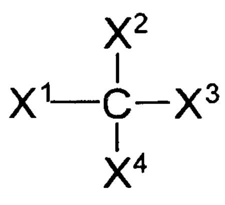

worin

$X^1$ einen der folgenden Reste bedeutet
$-L^1-R^{18}$, $-C(R^3)(R^4)-L^1-R^{18}$, $-L^1-C(R^3)(R^4)-R^{18}$, $-C(R^3)(R^4)-L^1-C(R^5)(R^6)-R^{18}$, $-L^1-Y-R^{18}$, $-Y-L^1-R^{18}$, $-Y-C(R^3)(R^4)-L^1-R^{18}$, $-C(R^3)(R^4)-Y-L^1-R^{18}$, $-C(R^3)(R^4)-L^1-Y-R^{18}$, $-Y-L^1-C(R^3)(R^4)-R^{18}$, $-L^1-Y-C(R^3)(R^4)-R^{18}$, $-L^1-C(R^3)(R^4)-Y-R^{18}$, $-Y-C(R^3)(R^4)-L^1-C(R^4)(R^6)-R^{18}$, $-C(R^3)(R^4)-Y-L^1-C(R^5)(R^6)-R^{18}$, $-C(R^3)(R^4)-L^1-Y-C(R^5)(R^6)-R^{18}$, $-C(R^3)(R^4)-L^1-C(R^5)(R^6)-Y-R^{18}$;
$X^2$ einen der folgenden Reste bedeutet $-R^7$, $(-CH_2-)_p-R^7$, $(-O-CH_2-)_p-R^7$;

$X^3$ einen der folgenden Reste bedeutet
$-M^1-R^{26}$, $-M^1-M^2-R^{26}$, $-M^1-(M^2)_r-M^3-R^{26}$, $-M^1-(M^2)_r-M^3-(M^4)_s-R^{26}$;
$X^4$ einen der folgenden Reste bedeutet
$-L^2-R^{19}$, $-C(R^{10})(R^{11})-L^2-R^{19}$, $-L^2-C(R^{10})(R^{11})-R^{19}$, $-C(R^{10})(R^{11})-C(R^{12})(R^{13})-L^2-R^{19}$, $-C(R^{10})(R^{11})-L^2-C(R^{12})(R^{13})-R^{19}$, $-L^2-C(R^{10})(R^{11})-C(R^{12})(R^{13})-R^{19}$;
$L^1$ für einen der folgenden Gruppen steht

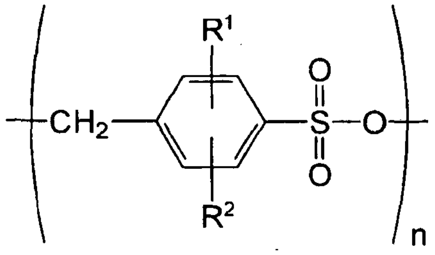
Linker 1

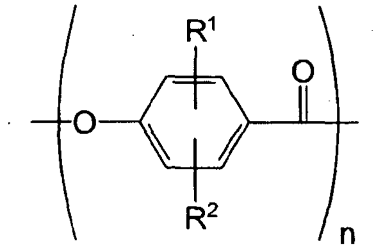
Linker 2

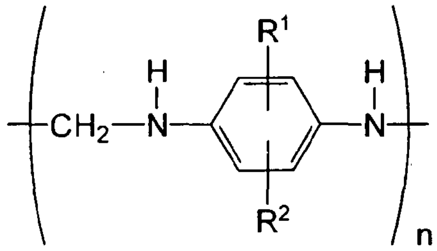
Linker 3

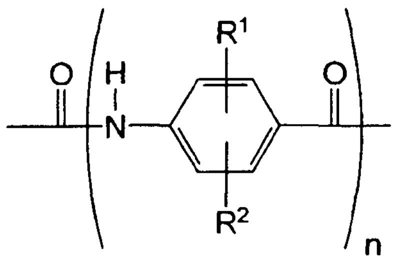
Linker 4

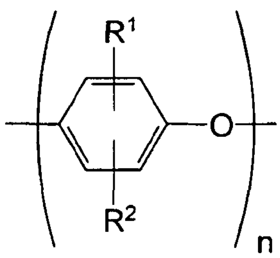
Linker 5

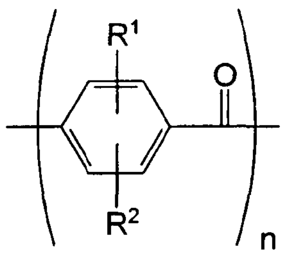
Linker 6

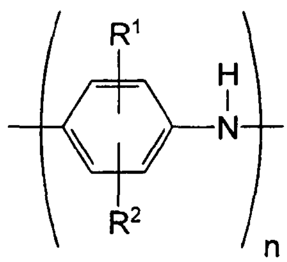
Linker 7

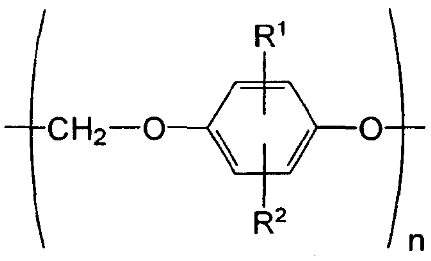
Linker 8

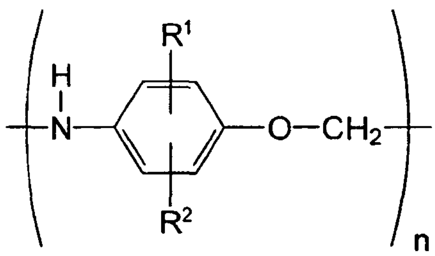
Linker 9

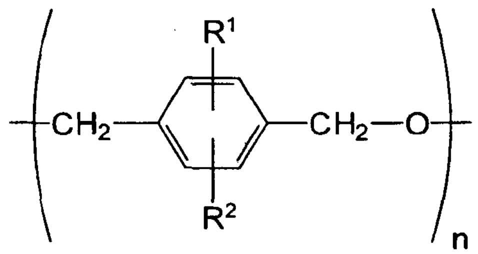

Linker 10

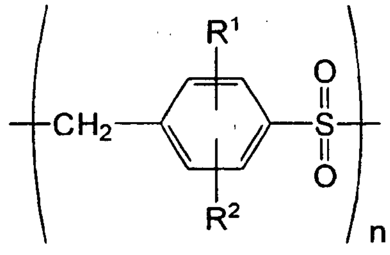

Linker 11

$L^2$ für einen der folgenden Gruppen steht
-O-CO- -NH-CO-, -CO-, -O-, -NH-, -CO-O-, -CO-NH-, -NH-CO-O-, -O-CO-NH-, -O-CO-O-, -NH-CO-NH-;
$M^1$ für einen der folgenden Gruppen steht

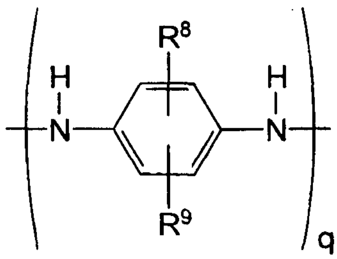

Gruppe 1

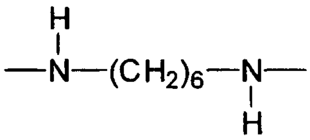

Gruppe 2

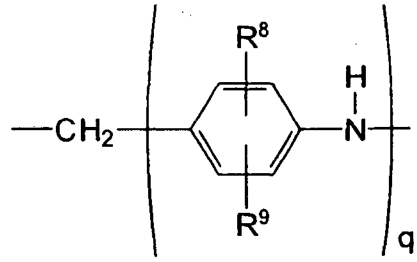

Gruppe 3

$M^2$ für einen der folgenden Gruppen steht
$-CH_2-$, $-CH_2-CH_2-$, $-CH_2-CH_2-CH_2-$, $-CH_2-CH_2-CH_2-CH_2-$, -O-, $-O-CH_2-$, $-O-CH_2-CH_2-$, $-O-CH_2-CH_2-CH_2-$, $-O-CH_2-CH_2-CH_2-CH_2-$, -O-CO-, $-O-CO-CH_2-$, $-O-CO-CH_2-CH_2-$, $-O-CO-CH_2-CH_2-CH_2-$, $-O-CO-CH_2-CH_2-CH_2-CH_2-$, -CO-, $-CO-CH_2-$, $-CO-CH_2-CH_2-$, $-CO-CH_2-CH_2-CH_2-$, $-CO-CH_2-CH_2-CH_2-CH_2-$;
$M^3$ für einen der folgenden Gruppen steht
eine Bindung, -NH-, -NH-CO-, -NH-CO-NH-, -NH-CS-, -NH-CS-NH-, -NH-C(NH)-NH-;
$M^4$ für einen der folgenden Gruppen steht
$(-CH_2-O-CH_2-)_t$, $(-O-CH_2-CH_2-)_t$, $(-CH_2-CH_2-O-)_t$;
Y für $(-CH_2-)_m$, $(-CH_2-O-)_m$, $(-O-CH_2-)_m$, $(-CH_2-CH_2-O-)_m$, $(-CH_2-CH_2-CH_2-O-)_m$;
$R^1$ bis $R^{13}$ unabhängig voneinander folgende Reste bedeuten: $-R^{14}$ bis $-R^{30}$, -OH, $-OCH_3$, $-OC_2H_5$, $-OC_3H_7$, $-O\text{-cyclo-}C_3H_5$, $-OCH(CH_3)_2$, $-OC(CH_3)_3$, $-OC_4H_9$, -OPh, $-OCH_2\text{-}Ph$, $-OCPh_3$, -SH, $-SCH_3$, $-SC_2H_5$, $-SC_3H_7$, $-S\text{-cyclo-}C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, $-CO\text{-cyclo-}C_3H_5$, $-COCH(CH_3)_2$, $-COC(CH_3)_3$, -COOH, -COCN, $-COOCH_3$, $-COOC_2H_5$, $-COOC_3H_7$, $-COO\text{-cyclo-}C_3H_5$, $-COOCH(CH_3)_2$, $-COOC(CH_3)_3$, $-O-CO-R^{14}$, $-CONH_2$, $-CONHCH_3$, $-CONHC_2H_5$, $-CONHC_3H_7$, $-CONH\text{-cyclo-}C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, $-CON(\text{cyclo-}C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, $-NHCO\text{-cyclo-}C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, $-NHCO-O\text{-cyclo-}C_3H_5$, $-NHCO-OCH(CH_3)_2$, $-NHCO-OC(CH_3)_3$, $-NH_2$, $-NHCH_3$, $-NHC_2H_5$, $-NHC_3H_7$, $-NH\text{-cyclo-}C_3H_5$, $-NHCH(CH_3)_2$, $-NHC(CH_3)_3$, $-N(CH_3)_2$, $-N(C_2H_5)_2$, $-N(C_3H_7)_2$, $-N(\text{cyclo-}C_3H_5)_2$, $-N[CH(CH_3)_2]_2$, $-N[C(CH_3)_3]_2$, $-SOCH_3$, $-SOC_2H_5$, $-SOC_3H_7$, $-SO\text{-cyclo-}C_3H_5$, $-SOCH(CH_3)_2$, $-SOC(CH_3)_3$, $-SO_2CH_3$, $-SO_2C_2H_5$, $-SO_2C_3H_7$, $-SO_2\text{-cyclo-}C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$,- $SO_3\text{-cyclo-}C_3H_5$, $-SO_3CH(CH_3)_2$, $-SO_3C(CH_3)_3$, $-SO_2NH_2$, $-OCF_3$, $-OC_2F_5$, $-O-COOCH_3$, $-O-COOC_2H_5$, $-O-COOC_3H_7$, $-O-COO\text{-cyclo-}C_3H_5$, $-O-COOCH(CH_3)_2$, $-O-COOC(CH_3)_3$, $-NH-CO-NH_2$, $-NH-CO-NHCH_3$, $-NH-CO-NHC_2H_5$, $-NH-CO-NHC_3H_7$, $-NH-CO-NH\text{-cyclo-}C_3H_5$, $-NH-CO-NH[CH(CH_3)_2]$, $-NH-CO-NH[C(CH_3)_3]$, $-NH-CO-N(CH_3)_2$, $-NH-CO-N(C_2H_5)_2$, $-NH-CO-N(C_3H_7)_2$, $-NH-CO-N(\text{cyclo-}C_3H_5)_2$, $-NH-CO-N[CH(CH_3)_2]_2$, $-NH-CO-N[C(CH_3)_3]_2$, $-NH-CS-NH_2$, $-NH-CS-NHCH_3$, $-NH-CS-NHC_2H_5$, $-NH-CS-NHC_3H_7$, $-NH-CS-NH\text{-cyclo-}C_3H_5$, $-NH-CS-NH[CH(CH_3)_2]$, $-NH-CS-NH[C(CH_3)_3]$, $-NH-CS-N(CH_3)_2$, $-NH-CS-N(C_2H_5)_2$, $-NH-CS-N(C_3H_7)_2$, $-NH-CS-N(\text{cyclo-}C_3H_5)_2$, $-NH-CS-N[CH(CH_3)_2]_2$, $-NH-CS-N[C(CH_3)_3]_2$, $-NH-C(=NH)-NH_2$, $-NH-C(=NH)-NHCH_3$, $-NH-C(=NH)-NHC_2H_5$, $-NH-C(=NH)-NHC_3H_7$, $-NH-C(=NH)-NH\text{-cyclo-}C_3H_5$, $-NH-C(=NH)-NH[CH(CH_3)_2]$, $-NH-C(=NH)-NH[C(CH_3)_3]$, $-NH-C(=NH)-N(CH_3)_2$, $-NH-C(=NH)-N(C_2H_5)_2$,

$-NH-C(=NH)-N(C_3H_7)_2$, $-NH-C(=NH)-N(cyclo-C_3H_5)_2$, $-NH-C(=NH)-N[CH(CH_3)_2]_2$, $-NH-C(=NH)-N[C(CH_2)_3]_2$, $-O-CO-NH_2$, $-O-CO-NHCH_3$, $-O-CO-NHC_2H_5$, $-O-CO-NHC_3H_7$, $-O-CO-NH-cyclo-C_3H_5$, $-O-CO-NH[CH(CH_3)_2]$, $-O-CO-NH[C(CH_3)_3]$, $-O-CO-N(CH_3)_2$, $-O-CO-N(C_2H_5)_2$, $-O-CO-N(C_3H_7)_2$, $-O-CO-N(cyclo-C_3H_5)_2$, $-O-CO-N[CH(CH_3)_2]_2$, $-O-CO-N[C(CH_3)_3]_2$, $-O-CO-OCH_3$, $-O-CO-OC_2H_5$, $-O-CO-OC_3H_7$, $-O-CO-O-cyclo-C_3H_5$, $-O-CO-OCH(CH_3)_2$, $-O-CO-OC(CH_3)_3$;
$R^{14}$ bis $R^{30}$ unabhängig voneinander folgende Reste bedeuten:

$-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, $cyclo-C_3H_5$, $cyclo-C_4H_7$, $cyclo-C_5H_9$, $cyclo-C_6H_{11}$, $cyclo-C_7H_{13}$, $cyclo-C_8H_{15}$, -Ph, $-CH_2-Ph$, $-CPh_3$, -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$, $-CH_2-CH=CH-CH=CH_2$,- $CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)=CH=CH_2$, $-CH_2-CH(CH_3)-CH_2-CH=CH_2$, $-CH(CH_3)-C_2H_4-CH=CH_2$, $-C_2H_4-CH=C(CH_3)_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH_2$, $-CH(CH_3)-CH_2-C(CH_3)=CH_2$, $-CH(CH_3)-CH(CH_3)-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[C(CH_3)_3]=CH_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-CH=CH-C_2H_4-CH=CH_2$, $-CH_2-CH=CH-CH=CH-CH_3$, $-CH=CH-CH_2-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CHr-C(CH_3)=CH-CH=CH_2$, $-CH(CH_3)-CH=CH-CH=CH_2$, $-CH=CH-CH_2-C(CH_3)=CH_2$, $-CH=CH-CH(CH_3)-CH=CH_2$, $-CH=C(CH_3)-CH_2-CH=CH_2$, $-C(CH_3)=CH-CH_2-CH=CH_2$, $-CH=CH-CH=C(CH_3)_2$, $-CH=CH-C(CH_3)=CH-CH_3$, $-CH=C(CH_3)-CH=CH-CH_3$, $-C(CH_3)=CH-CH=CH-CH_3$, $-CH=C(CH_3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-CH_2-CH(CH_3)-C\equiv CH$, $-CH(CH_3)-CH_2-C\equiv CH$, $-CH(CH_3)-C\equiv -C-CH_3$, $-C_4H_8-C\equiv CH$, $-C_3H_6-C\equiv C-CH_3$, $-C_2H_4-C\equiv C-C_2H_5$; $-CH_2-C\equiv C-C_3H_7$, $-C\equiv C-C_4H_9$, $-C_2H_4-CH(CH_3)-C\equiv CH$, $-CH_2-CH(CH_3)-CH_2-C\equiv CH$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$,- $C\equiv C-CH_2-CH(CH_3)_2$, $-C\equiv C-C(CH_3)_3$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)(C_2H_5)-C\equiv CH$, $-C\equiv C-C\equiv CH$, $-CH_2-C\equiv C-C\equiv CH$, $-C\equiv C-C\equiv C-CH_3$, $-CH(C\equiv CH)_2$, $-C_2H_4-C\equiv C-C\equiv CH$, $-CH_2-C\equiv C-CH_2-C\equiv CH$, $-C\equiv C-C_2H_4-C\equiv CH$, $-CH_2-C\equiv C-C\equiv C-CH_3$, $-C\equiv C-CH_2-C\equiv C-CH_3$, $-C\equiv C-C\equiv C-C_2H_5$, $-C\equiv C-CH(CH_3)-C\equiv CH$, $-CH(CH_3)-C\equiv C-C\equiv CH$, $-CH(C\equiv CH)-CH_2-C\equiv CH$, $-C(C\equiv CH)_2-CH_3$, $-CH_2-CH(C\equiv CH)_2$, $-CH(C\equiv CH)-C\equiv C-CH_3$;

m ist eine ganze Zahl von 1 bis 10;
n ist eine ganze Zahl von 0 bis 5;
p ist eine ganze Zahl von 0 bis 3;
q ist eine ganze Zahl von 0 bis 4;
r steht für 0 oder 1;
s steht für 0 oder 1;
t ist eine ganze Zahl von 1 bis 10.

**2.** Katheterballon gemäß Anspruch 1, wobei der mindestens eine antirestenotische Wirkstoff in dem mindestens einen transportfördernden Molekulardispersionsmittel eingebettet oder eingelagert ist.

**3.** Katheterballon gemäß Anspruch 1 oder 2, wobei das Mengenverhältnis von antirestenotischen Wirkstoff und dem mindestens einen transportfördernden Molekulardispersionsmittel von 90 Gew.-% antirestenotischen Wirkstoff zu 10 Gew.-% transportfördernden Molekulardispersionsmittel bis 10 Gew.-% antirestenotischen Wirkstoff zu 90 Gew.-% transportfördernden Molekulardispersionsmittel beträgt.

**4.** Katheterballon gemäß einem der Ansprüche 1 - 3, wobei der mindestens eine antirestenotische Wirkstoff ausgewählt wird aus der Gruppe umfassend oder bestehend aus: Paclitaxel, Docetaxel, Sirolimus, Biolimus A9, Zotarolimus, Everolimus; Myolimus, Novolimus, Pimecrolimus, Tacrolimus, Ridaforolimus; und Temsirolimus.

**5.** Katheterballon gemäß Anspruch 4, wobei es sich bei dem mindestens einen antirestenotischen Wirkstoff um Paclitaxel handelt.

**6.** Katheterballon gemäß Anspruch 4, wobei es sich bei dem mindestens einen antirestenotischen Wirkstoff um Sirolimus handelt.

**7.** Katheterballon gemäß einem der Ansprüche 1 - 6, wobei die Verbindung gemäß allgemeiner Formel (I) einen Siedepunkt von mindestens 500°C (bei Normaldruck) besitzt.

**8.** Katheterballon gemäß einem der Ansprüche 1 - 7, wobei die Verbindung gemäß allgemeiner Formel (I) 7 bis 9 Sauerstoffatome enthält.

**9.** Katheterballon gemäß einem der Ansprüche 1 - 8, wobei die Verbindung gemäß allgemeiner Formel (I) eine molare Masse (Molekulargewicht) von mindestens 500 g/mol besitzt.

**10.** Katheterballon gemäß einem der Ansprüche 1 - 9, wobei die Verbindung gemäß allgemeiner Formel (I) einen Schmelzpunkt oberhalb von -60°C besitzt.

**11.** Katheterballon gemäß einem der Ansprüche 1 - 10, wobei die Verbindung gemäß allgemeiner Formel (I) eine Dichte von 0,95 $g/cm^3$ bis 1,05 $g/cm^3$ besitzt.

**12.** Katheterballon gemäß einem der Ansprüche 1 - 11, wobei die Verbindung gemäß allgemeiner Formel (I) einen Flammpunkt oberhalb von 100°C besitzt.

**13.** Katheterballon gemäß einem der Ansprüche 1 - 12, wobei die Verbindung gemäß allgemeiner Formel (I) einen Brechungsindex $n_D^{20}$ zwischen 1,440 und 1,460 besitzt.

**14.** Verfahren zur Herstellung eines Katheterballons gemäß einem der Ansprüche 1 - 13, umfassend die folgenden Schritte:

a) Bereitstellen eines Katheterballons eines Ballonkatheters,
b) Bereitstellen einer Beschichtungslösung aus dem mindestens einen antirestenotischen Wirkstoff und dem mindestens einen transportfördernden Molekulardispersionsmittel in einem Lösungsmittel oder Lösungsmittelgemisch,
c) Beschichten des Katheterballons mit der Beschichtungslösung mittels Tauch-, Streich-, Sprüh-, Pinsel- oder Pipettierverfahren
d) Trocknung der aufgebrachten Beschichtung.

**Claims**

**1.** Catheter balloon with or without crimped stent, wherein the surface of the catheter balloon is coated at least partially with at least one anti-restenotic agent and at least one transport promoting molecular dispersant, wherein the at least one transport promoting molecular dispersant is at least one compound of general formula (I):

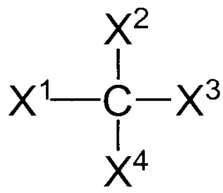

wherein

$X^1$ represents one of the following residues $-L^1-R^{18}$, $-C(R^3)(R^4)-L^1-R^{18}$, $-L^1-C(R^3)(R^4)-R^{18}$, $-C(R^3)(R^4)-L^1-C(R^5)(R^6)-R^{18}$, $-L^1-Y-R^{18}$, $-Y-L^1-R^{18}$, $-Y-C(R^3)(R^4)-L^1-R^{18}$, $-C(R^3)(R^4)-Y-L^1-R^{18}$, $-C(R^3)(R^4)-L^1-Y-R^{18}$, $-Y-L^1-C(R^3)(R^4)-R^{18}$, $-L^1-Y-C(R^3)(R^4)-R^{18}$, $-L^1-C(R^3)(R^4)-Y-R^{18}$, $-Y-C(R^3)(R^4)-L^1-C(R^5)(R^6)-R^{18}$, $-C(R^3)(R^4)-Y-L^1-C(R^5)(R^6)-R^{18}$, $-C(R^3)(R^4)-L^1-Y-C(R^5)(R^6)-R^{18}$, $-C(R^3)(R^4)-L^1-C(R^5)(R^6)-Y-R^{18}$;

$X^2$ represents one of the following residues $-R^7$, $(-CH_2-)_p-R^7$, $(-O-CH_2-)_p-R^7$;

$X^3$ represents one of the following residues $-M^1-R^{26}$, $-M^1-M^2-R^{26}$, $-m^1-(m^2)_r-M^3-R^{26}$, $-M^1-(M^2)_r-M^3-(M^4)_s-R^{26}$;

$X^4$ represents one of the following residues $-L^2-R^{19}$, $-C(R^{10})(R^{11})-L^2-R^{19}$, $-L^2-C(R^{10})(R^{11})-R^{19}$, $-C(R^{10})(R^{11})-C(R^{12})(R^{13})-L^2-R^{19}$, $-C(R^{10})(R^{11})-L^2-C(R^{12})(R^{13})-R^{19}$, $-L^2-C(R^{10})(R^{11})-C(R^{12})(R^{13})-R^{19}$;

$L^1$ represents one of the following groups

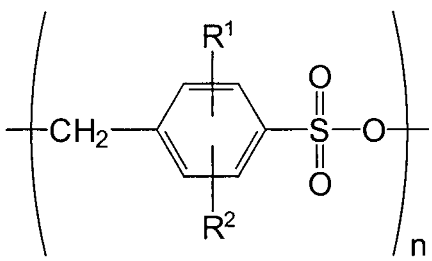

Linker 1

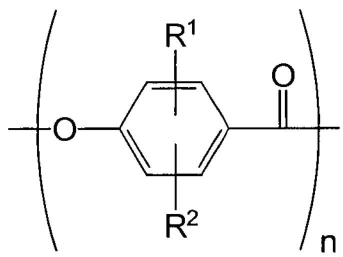

Linker 2

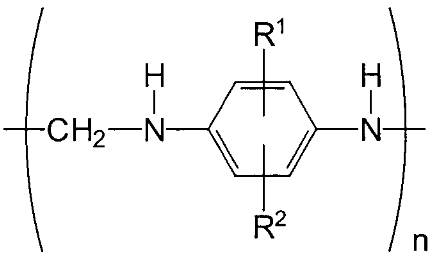

Linker 3

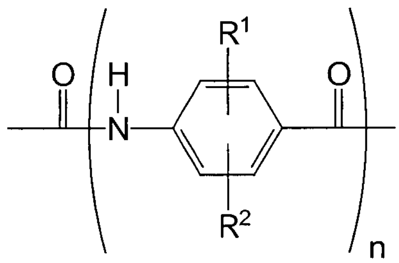

Linker 4

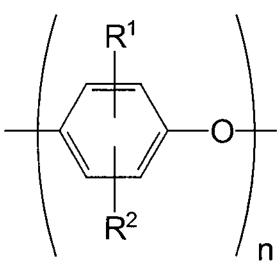

Linker 5

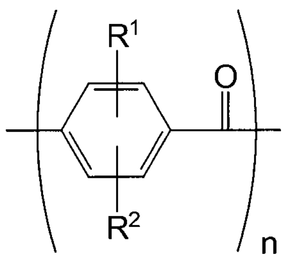

Linker 6

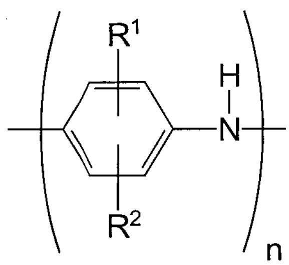

Linker 7

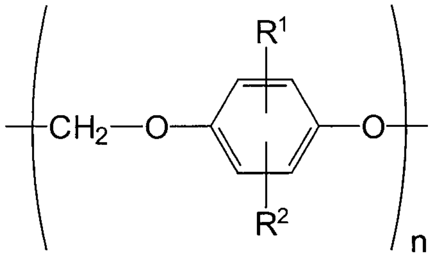

Linker 8

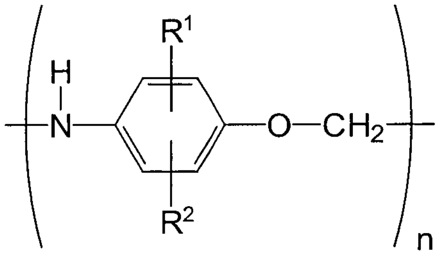

Linker 9

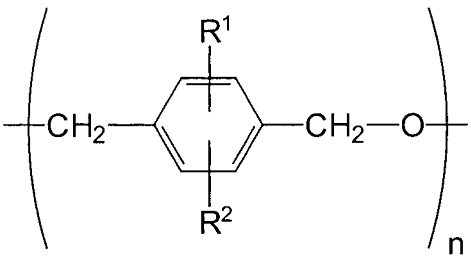

Linker 10

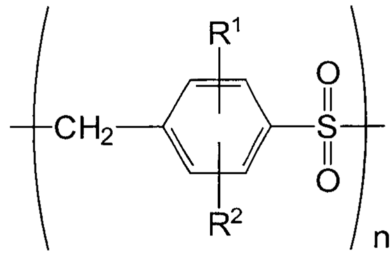

Linker 11

$L^2$ represents one of the following groups
-O-CO-, -NH-CO-, -CO-, -O-, -NH-, -CO-O-, -CO-NH-, -NH-CO-O-, -O-CO-NH-, -O-CO-O-, -N H-CO-N H-;
$M^1$ represents one of the following groups

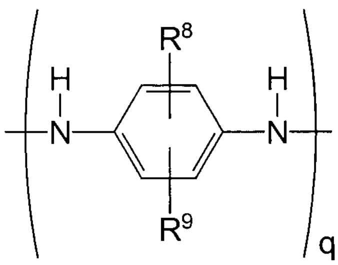

Group 1

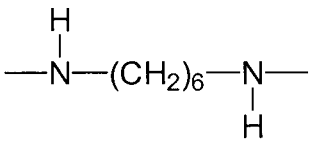

Group 2

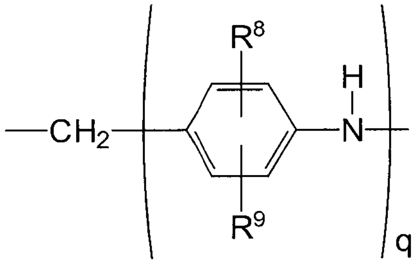

Group 3

$M^2$ represents one of the following groups
$-CH_2-$, $-CH_2-CH_2-$, $-CH_2-CH_2-CH_2-$, $-CH_2-CH_2-CH_2-CH_2-$, -O-, $-O-CH_2-$, $-O-CH_2-CH_2-$, $-O-CH_2-CH_2-CH_2-$, $-O-CH_2-CH_2-CH_2-CH_2-$, -O-CO-, $-O-CO-CH_2-$, $-O-CO-CH_2-CH_2-$, $-O-CO-CH_2-CH_2-CH_2-$, $-O-CO-CH_2-CH_2-CH_2-CH_2-$, -CO-, $-CO-CH_2-$, $-CO-CH_2-CH_2-$, $-CO-CH_2-CH_2-CH_2-$, $-CO-CH_2-CH_2-CH_2-CH_2-$;
$M^3$ represents one of the following groups
a bond, -NH-, -NH-CO-, -NH-CO-NH-, -NH-CS-, -NH-CS-NH-, -NH-C(NH)-NH-;
$M^4$ represents one of the following groups $(-CH_2-O-CH_2-)_t$, $(-O-CH_2-CH_2-)_t$, $(-CH_2-CH_2-O-)_t$;
Y represents $(-CH_2-)_m$, $(-CH_2-O-)_m$, $(-O-CH_2-)_m$, $(-CH_2-CH_2-O-)_m$, $(-CH_2-CH_2-CH_2-O-)_m$;
$R^1$ to $R^{13}$ represent independently of each other the following residues: $-R^{14}$ to $-R^{30}$, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2-Ph$, $-OCPh_3$, -SH, $-SCH_3$, $-SC_2H_5$, $-SC_3H_7$, -S-cyclo-$C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, -COCN, $-COOCH_3$, $-COOC_2H_5$, $-COOC_3H_7$, -COO-cyclo-$C_3H_5$, $-COOCH(CH_3)_2$, $-COOC(CH_3)_3$, $-O-CO-R^{14}$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, -NHCO-O-cyclo-$C_3H_5$, $-NHCO-OCH(CH_3)_2$, $-NHCO-OC(CH_3)_3$, $-NH_2$,

$-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $-SOCH_3$, $-SOC_2H_5$, $-SOC_3H_7$, -SO-cyclo-$C_3H_5$, $-SOCH(CH_3)_2$, $-SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$,- $SO_3$-cyclo-$C_3H_5$, $-SO_3CH(CH_3)_2$, $-SO_3C(CH_3)_3$, $-SO_2NH_2$, $-OCF_3$, $-OC_2F_5$, $-O-COOCH_3$, $-O-COOC_2H_5$, $-O-COOC_3H_7$, -O-COO-cyclo-$C_3H_5$, $-O-COOCH(CH_3)_2$, $-O-COOC(CH_3)_3$, $-NH-CO-NH_2$, $-NH-CO-NHCH_3$, $-NH-CO-NHC_2H_5$, $-NH-CO-NHC_3H_7$, -NH-CO-NH-cyclo-$C_3H_5$, $-NH-CO-NH[CH(CH_3)_2]$, $-NH-CO-NH[C(CH_3)_3]$, $-NH-CO-N(CH_3)_2$, $-NH-CO-N(C_2H_5)_2$, $-NH-CO-N(C_3H_7)_2$, -NH-CO-N(cyclo-$C_3H_5)_2$, $-NH-CO-N[CH(CH_3)_2]_2$, $-NH-CO-N[C(CH_3)_3]_2$, $-NH-CS-NH_2$, $-NH-CS-NHCH_3$, $-NH-CS-NHC_2H_5$, $-NH-CS-NHC_3H_7$, -NH-CS-NH-cyclo-$C_3H_5$, $-NH-CS-NH[CH(CH_3)_2]$, $-NH-CS-NH[C(CH_3)_3]$, $-NH-CS-N(CH_3)_2$, $-NH-CS-N(C_2H_5)_2$, $-NH-CS-N(C_3H_7)_2$, -NH-CS-N(cyclo-$C_3H_5)_2$, $-NH-CS-N[CH(CH_3)_2]_2$, $-NH-CS-N[C(CH_3)_3]_2$, $-NH-C(=NH)-NH_2$, $-NH-C(=NH)-NHCH_3$, $-NH-C(=NH)-NHC_2H_5$, $-NH-C(=NH)-NHC_3H_7$, -NH-C(=NH)-NH-cyclo-$C_3H_5$, $-NH-C(=NH)-NH[CH(CH_3)_2]$, $-NH-C(=NH)-NH[C(CH_3)_3]$, $-NH-C(=NH)-N(CH_3)_2$, $-NH-C(=NH)-N(C_2H_5)_2$, $-NH-C(=NH)-N(C_3H_7)_2$, -NH-C(=NH)-N(cyclo-$C_3H_5)_2$, $-NH-C(=NH)-N[CH(CH_3)_2]_2$, $-NH-C(=NH)-N[C(CH_3)_3]_2$, $-O-CO-NH_2$, $-O-CO-NHCH_3$, $-O-CO-NHC_2H_5$, $-O-CO-NHC_3H_7$, -O-CO-NH-cyclo-$C_3H_5$, $-O-CO-NH[CH(CH_3)_2]$, $-O-CO-NH[C(CH_3)_3]$, $-O-CO-N(CH_3)_2$, $-O-CO-N(C_2H_5)_2$, $-O-CO-N(C_3H_7)_2$, -O-CO-N(cyclo-$C_3H_5)_2$, $-O-CO-N[CH(CH_3)_2]_2$, $-O-CO-N[C(CH_3)_3]_2$, $-O-CO-OCH_3$, $-O-CO-OC_2H_5$, $-O-CO-OC_3H_7$, -O-CO-O-cyclo-$C_3H_5$, $-O-CO-OCH(CH_3)_2$, $-O-CO-OC(CH_3)_3$;

$R^{14}$ to $R^{30}$ represent independently of each other the following residues:

$-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_3H_5$, cyclo-$C_4H_7$, cyclo-$C_5H_9$, cyclo-$C_6H_{11}$, cyclo-$C_7H_{13}$, cyclo-$C_8H_{15}$, -Ph, $-CH_2$-Ph, $-CPh_3$, -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$,- $CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-CH(CH_3)-C_2H_4-CH=CH_2$, $-C_2H_4-CH=C(CH_3)_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)-C_2H_5$, $-CH=C(CH_3)-C_3H_7$, $-C(CH_3)=CH-C_3H_7$, $-CH_2-CH(CH_3)-C(CH_3)=CH2$, $-CH(CH_3)-CH2-C(CH_3)=CH_2$, $-CH(CH_3)-CH(CH_3)-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, $-CH(CH_3)-C(CH_3)=CH-CH_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)(C_2H_5)-CH=CH_2$, $-CH(CH_3)-C(C_2H_5)=CH_2$, $-CH_2-C(C_3H_7)=CH_2$, $-CH_2-C(C_2H_5)=CH-CH_3$, $-CH(C_2H_5)-CH=CH-CH_3$, $-C(C_4H_9)=CH_2$, $-C(C_3H_7)=CH-CH_3$, $-C(C_2H_5)=CH-C_2H_5$, $-C(C_2H_5)=C(CH_3)_2$, $-C[C(CH_3)_3]=CH_2$, $-C[CH(CH_3)(C_2H_5)]=CH_2$, $-C[CH_2-CH(CH_3)_2]=CH_2$, $-C_2H_4-CH=CH-CH=CH_2$, $-CH_2-CH=CH-CH_2-CH=CH_2$, $-CH=CH-C_2H_4-CH=CH_2$, $-CH_2-CH=CH-CH=CH-CH_3$, $-CH=CH-CH_2-CH=CH-CH_3$, $-CH=CH-CH=CH-C_2H_5$, $-CH_2-CH=CH-C(CH_3)=CH_2$, $-CH_2-CH=C(CH_3)-CH=CH_2$, $-CH_2-C(CH_3)=CH-CH=CH_2$, $-CH(CH_3)-CH=CH-CH=CH_2$, $-CH=CH-CH_2-C(CH_3)=CH_2$, $-CH=CH-CH(CH_3)-CH=CH_2$, $-CH=C(CH_3)-CH_2-CH=CH_2$, $-C(CH_3)=CH-CH_2-CH=CH_2$, $-CH=CH-CH=C(CH_3)_2$, $-CH=CH-C(CH_3)=CH-CH_3$, $-CH=C(CH_3)-CH=CH-CH_3$, $-C(CH_3)=CH-CH=CH-CH_3$, $-CH=C(CH3)-C(CH_3)=CH_2$, $-C(CH_3)=CH-C(CH_3)=CH_2$, $-C(CH_3)=C(CH_3)-CH=CH_2$, $-CH=CH-CH=CH-CH=CH_2$, $-C\equiv CH$, $-C\equiv C-CH_3$, $-CH_2-C\equiv CH$, $-C_2H_4-C\equiv CH$, $-CH_2-C\equiv C-CH_3$, $-C\equiv C-C_2H_5$, $-C_3H_6-C\equiv CH$, $-C_2H_4-C\equiv C-CH_3$, $-CH_2-C\equiv C-C_2H_5$, $-C\equiv C-C_3H_7$, $-CH(CH_3)-C\equiv CH$, $-CH_2-CH(CH_3)-C\equiv CH$, $-CH(CH_3)-CH_2-C\equiv CH$,- $CH(CH_3)-C\equiv C-CH_3$, $-C_4H_8-C\equiv CH$, $-C_3H_6-C\equiv C-CH_3$, $-C_2H_4-C\equiv C-C_2H_5$, $-CH_2-C\equiv C-C_3H_7$, $-C\equiv C-C_4H_9$, $-C_2H_4-CH(CH_3)-C\equiv CH$, $-CH_2-CH(CH_3)-CH_2-C\equiv CH$, $-CH(CH_3)-C_2H_4-C\equiv CH$, $-CH_2-CH(CH_3)-C\equiv C-CH_3$, $-CH(CH_3)-CH_2-C\equiv C-CH_3$, $-CH(CH_3)-C\equiv C-C_2H_5$, $-CH_2-C\equiv C-CH(CH_3)_2$, $-C\equiv C-CH(CH_3)-C_2H_5$, $-C\equiv C-CH_2-CH(CH_3)_2$, $-C\equiv C-C(CH_3)_3$, $-CH(C_2H_5)-C\equiv C-CH_3$, $-C(CH_3)_2-C\equiv C-CH_3$, $-CH(C_2H_5)-CH_2-C\equiv CH$, $-CH_2-CH(C_2H_5)-C\equiv CH$, $-C(CH_3)_2-CH_2-C\equiv CH$, $-CH_2-C(CH_3)_2-C\equiv CH$, $-CH(CH_3)-CH(CH_3)-C\equiv CH$, $-CH(C_3H_7)-C\equiv CH$, $-C(CH_3)$

$(C_2H_5)$-C≡CH, -C≡C-C≡CH, -$CH_2$-C≡C-C≡CH, -C≡C-C≡C-$CH_3$, -CH(C≡CH)$_2$, -$C_2H_4$-C≡C-C≡CH, -$CH_2$-C≡C-$CH_2$-C≡CH, -C≡C-$C_2H_4$-C≡CH, -$CH_2$-C≡C-C≡C-$CH_3$, -C≡C-$CH_2$-C≡C-$CH_3$, -C≡C-C≡C-$C_2H_5$, -C≡C-CH($CH_3$)-C≡CH, -CH($CH_3$)-C≡C-C≡CH, -CH(C≡CH)-$CH_2$-C≡CH, -C(C≡CH)$_2$-$CH_3$, -$CH_2$-CH (C≡CH)$_2$, -CH(C≡CH)-C≡C-$CH_3$;

m is an integer from 1 to 10;
n is an integer from 0 to 5;
p is an integer from 0 to 3;
q is an integer from 0 to 4;
r represents 0 or 1;
s represents 0 or 1;
t is an integer from 1 to 10.

**2.** The catheter balloon according to claim 1, wherein the at least one anti-restenotic agent is embedded or stored in the at least one transport promoting molecular dispersant.

**3.** The catheter balloon according to claim 1 or 2, wherein the ratio of the anti-restenotic agent and the at least one transport promoting molecular dispersant is from 90 wt % anti-restenotic agent to 10 wt % transport promoting molecular dispersant to 10 wt % anti-restenotic agent to 90 wt % transport promoting molecular dispersant.

**4.** The catheter balloon according to one of the claims 1-3, wherein the at least one anti-restenotic agent is selected from the group comprising or consisting of: paclitaxel, docetaxel, sirolimus, biolimus A9, zotarolimus, everolimus, myolimus, novolimus, pimecrolimus, tacrolimus, ridaforolimus, and temsirolimus.

**5.** The catheter balloon according to claim 4, wherein the at least one anti-restenotic agent is paclitaxel.

**6.** The catheter balloon according to claim 4, wherein the at least one anti-restenotic agent is sirolimus.

**7.** The catheter balloon according to one of the claims 1 - 6, wherein the compound according to general formula (I) has a boiling point of at least 500°C (at atmospheric pressure).

**8.** The catheter balloon according to one of the claims 1 - 7, wherein the compound according to general formula (I) contains 7 to 9 oxygen atoms.

**9.** The catheter balloon according to one of the claims 1 - 8, wherein the compound according to general formula (I) has a molar mass (molecular weight) of at least 500 g/mol.

**10.** The catheter balloon according to one of the claims 1 - 9, wherein the compound according to general formula (I) has a melting point of above -60°C.

**11.** The catheter balloon according to one of the claims 1 - 10, wherein the compound according to general formula (I) has a density from 0.95 g/cm$^3$ to 1.05 g/cm$^3$.

**12.** The catheter balloon according to one of the claims 1 - 11, wherein the compound according to general formula (I) has a flash point of above100°C.

**13.** The catheter balloon according to one of the claims 1 - 12, wherein the compound according to general formula (I) has a refractive index $n_D^{20}$ between 1.440 and 1.460

**14.** A method of producing of a catheter balloon according to one of the claims 1-13 comprising the steps of:

a) providing a catheter balloon of a balloon catheter,
b) providing a coating solution of the at least one anti-restenotic agent and the at least one transport promoting molecular dispersant in a solvent or solvent mixture,
c) coating of the catheter balloon with the coating solution by means of dipping, spreading, spraying, brushing or pipetting procedure,
d) drying the applied coating.

## Revendications

**1.** Un ballonnet à cathéter avec ou sans un stent serti sur le cathéter, dans lequel la surface du ballonnet à cathéter est au moins partiellement revêtue d'au moins un agent anti-sténotique et d'au moins un agent dispersant moléculaire pour promouvoir le transport, dans lequel l'au moins un agent dispersant moléculaire pour promouvoir le transport est un composé de formule générale (I)

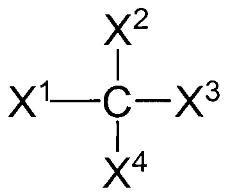

dans laquelle

$X^1$ représente l'un des résidus suivants
$-L^1-R^{18}$, $-C(R^3)(R^4)-L^1-R^{18}$, $-L^1-C(R^3)(R^4)-R^{18}$, $-C(R^3)(R^4)-L^1-C(R^5)(R^6)-R^{18}$, $-L^1-Y-R^{18}$, $-Y-L^1-R^{18}$, $-Y-C(R^3)(R^4)-L^1-R^{18}$, $-C(R^3)(R^4)-Y-L^1-R^{18}$, $-C(R^3)(R^4)-L^1-Y-R^{18}$, $-Y-L^1-C(R^3)(R^4)-R^{18}$, $-L^1-Y-C(R^3)(R^4)-R^{18}$, $-L^1-C(R^3)(R^4)-Y-R^{18}$, $-Y-C(R^3)(R^4)-L^1-C(R-)(R^6)-R^{18}$, $-C(R^3)(R^4)-Y-L^1-C(R^5)(R^6)-R^{18}$, $-C(R^3)(R^4)-L^1-Y-C(R^5)(R^6)-R^{18}$, $-C(R^3)(R^4)-L^1-C(R^5)(R^6)-Y-R^{18}$;
$X^2$ représente l'un des résidus suivants $-R^7$, $(-CH_2-)_p-R^7$, $(-O-CH_2-)_p-R^7$;
$X^3$ représente l'un des résidus suivants $-M^1-R^{26}$, $-M^1-M^2-R^{26}$, $-M^1-(M^2)_r-M^3-R^{26}$, $-M^1-(M^2)_r-M^3-(M^4)_s-R^{26}$;
$X^4$ représente l'un des résidus suivants
$-L^2-R^{19}$, $-C(R^{10})(R^{11})-L^2-R^{19}$, $-L^2-C(R^{10})(R^{11})-R^{19}$, $-C(R^{10})(R^{11})-C(R^{12})(R^{13})-L^2-R^{19}$, $-C(R^{10})(R^{11})-L^2-C(R^{12})(R^{13})-R^{19}$, $-L_2-C(R^{10})(R^{11})-C(R^{12})(R^{13})-R^{19}$;
$L^1$ représente l'un des groupes suivants

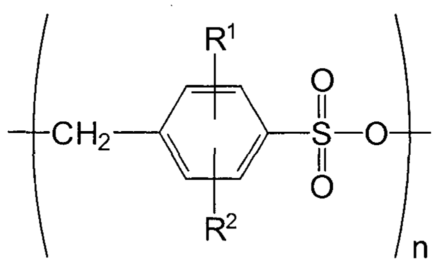

Linker 1

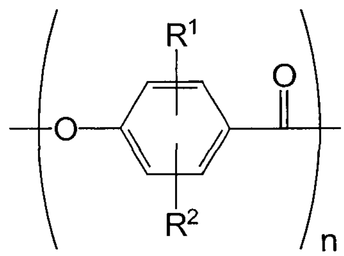

Linker 2

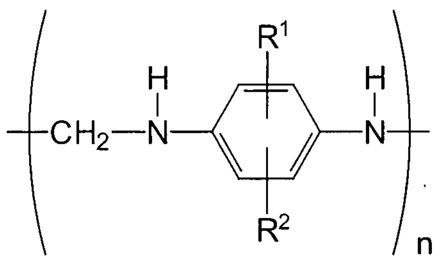

Linker 3

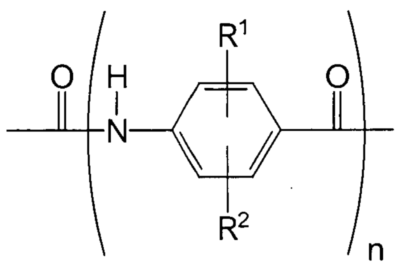

Linker 4

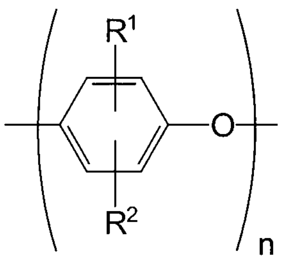

Linker 5

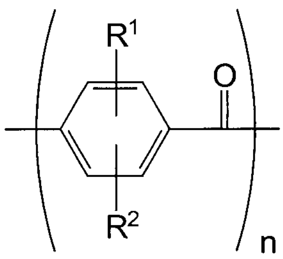

Linker 6

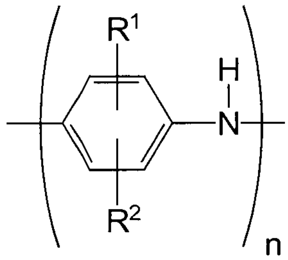

Linker 7

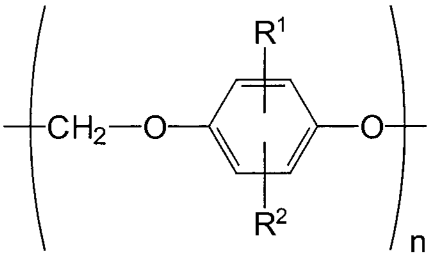

Linker 8

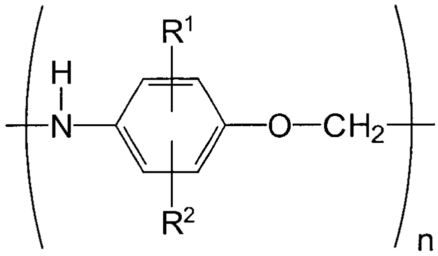

Linker 9

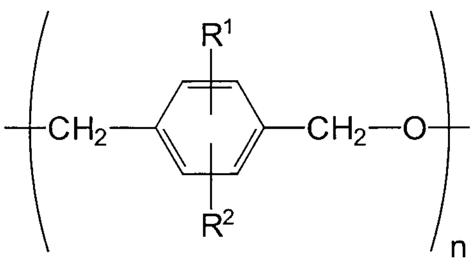

Linker 10

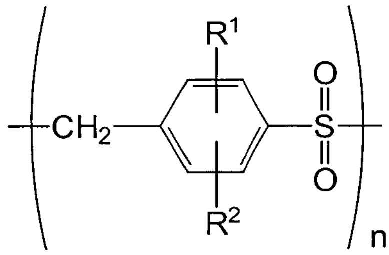

Linker 11

$L^2$ représente l'un des groupes suivants
-O-CO-, -NH-CO-, -CO-, -O-, -NH-, -CO-O-, -CO-NH-, -N H-C O-O-, -O-CO-NH-, -O-CO-O-, -NH-CO-NH-;
$M^1$ représente l'un des groupes suivants

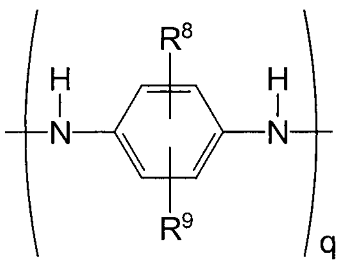

Groupe 1

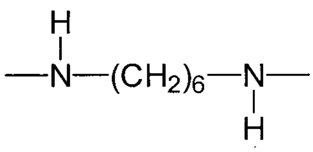

Groupe 2

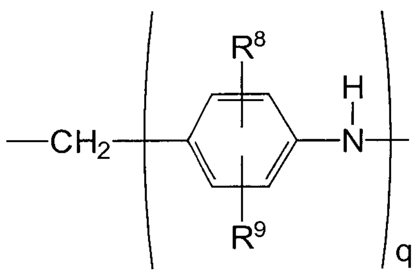

Groupe 3

$M^2$ représente l'un des groupes suivants
$-CH_2-$, $-CH_2-CH_2-$, $-CH_2-CH_2-CH_2-$, $-CH_2-CH_2-CH_2-CH_2-$, -O-, $-O-CH_2-$, $-O-CH_2-CH_2-$, $-O-CH_2-CH_2-CH_2-$, $-O-CH_2-CH_2-CH_2-CH_2-$, -O-CO-, $-O-CO-CH_2-$, $-O-CO-CH_2-CH_2-$, -O-CO-C $H_2$-$CH_2$-CH2-, $-O-CO-CH_2-CH_2-CH_2-CH_2-$, -CO-, $-CO-CH_2-$, $-CO-CH_2-CH_2-$, $-CO-CH_2-CH_2-CH_2-$, $-CO-CH_2-CH_2-CH_2-$CH2-;
$M^3$ représente l'un des groupes suivants
une liaison, -NH-, -NH-CO-, -NH-CO-NH-, -NH-CS-, -NH-CS-NH-, -NH-C(NH)-NH-;

$M^4$ représente l'un des groupes suivants
$(-CH_2-O-CH_2-)t$, $(-O-CH_2-CH_2-)_t$, $(-CH_2-CH_2-O-)_t$;
Y représente $(-CH_2-)_m$, $(-CH_2-O-)_m$, $(-O-CH_2-)_m$, $(-CH_2-CH_2-O-)_m$, $(-CH_2-CH_2-CH_2-O-)_m$;
$R^1$ jusqu'à $R^{13}$ représentent indépendamment l'un de l'autre les résidus suivants:

$-R^{14}$ jusqu' à $-R^{30}$, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-SC_3H_7$, -S-cyclo-$C_3H_5$, $-SCH(CH_3)_2$, $-SC(CH_3)_3$, $-NO_2$, -F, -Cl, -Br, -I, $-P(O)(OH)_2$, $-P(O)(OCH_3)_2$, $-P(O)(OC_2H_5)_2$, $-P(O)(OCH(CH_3)_2)_2$, $-C(OH)[P(O)(OH)_2]_2$, $-Si(CH_3)_2(C(CH_3)_3)$, $-Si(C_2H_5)_3$, $-Si(CH_3)_3$, $-N_3$, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, -COCN, $-COOCH_3$, $-COOC_2H_5$, $-COOC_3H_7$, -COO-cyclo-$C_3H_5$, $-COOCH(CH_3)_2$, $-COOC(CH_3)_3$, $-O-CO-R^{14}$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, -NHCO-O-cyclo-$C_3H_5$, $-NHCO-OCH(CH_3)_2$, $-NHCO-OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, $-SOCH_3$, $-SOC_2H_5$, $-SOC_3H_7$, -SO-cyclo-$C_3H_5$, $-SOCH(CH_3)_2$, $-SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C(CH_3)_3$, $-SO_3H$, $-SO_3CH_3$, $-SO_3C_2H_5$, $-SO_3C_3H_7$,- $SO_3$-cyclo-$C_3H_5$, $-SO_3CH(CH_3)_2$, $-SO_3C(CH_3)_3$, $-SO_2NH_2$, $-OCF_3$, $-OC_2F_5$, $-O-COOCH_3$, $-O-COOC_2H_5$, $-O-COOC_3H_7$, -O-COO-cyclo-$C_3H_5$, $-O-COOCH(CH_3)_2$, $-O-COOC(CH_3)_3$, $-NH-CO-NH_2$, $-NH-CO-NHCH_3$, $-NH-CO-NHC_2H_5$, $-NH-CO-NHC_3H_7$, -NH-CO-NH-cyclo-$C_3H_5$, $-NH-CO-NH[CH(CH_3)_2]$, $-NH-CO-NH[C(CH_3)_3]$, $-NH-CO-N(CH_3)_2$, $-NH-CO-N(C_2H_5)_2$, $-NH-CO-N(C_3H_7)_2$, -NH-CO-N(cyclo-$C_3H_5)_2$, $-NH-CO-N[CH(CH_3)_2]_2$, $-NH-CO-N[C(CH_3)_3]_2$, $-NH-CS-NH_2$, $-NH-CS-NHCH_3$, $-NH-CS-NHC_2H_5$, $-NH-CS-NHC_3H_7$, -NH-CS-NH-cyclo-$C_3H_5$, $-NH-CS-NH[CH(CH_3)_2]$, $-NH-CS-NH[C(CH_3)_3]$, $-NH-CS-N(CH_3)_2$, $-NH-CS-N(C_2H_5)_2$, $-NH-CS-N(C_3H_7)_2$, -NH-CS-N(cyclo-$C_3H_5)_2$, $-NH-CS-N[CH(CH_3)_2]_2$, $-NH-CS-N[C(CH_3)_3]_2$, $-NH-C(=NH)-NH_2$, $-NH-C(=NH)-NHCH_3$, $-NH-C(=NH)-NHC_2H_5$, $-NH-C(=NH)-NHC_3H_7$, -NH-C(=NH)-NH-cyclo-$C_3H_5$, $-NH-C(=NH)-NH[CH(CH_3)_2]$, $-NH-C(=NH)-NH[C(CH_3)_3]$, $-NH-C(=NH)-N(CH_3)_2$, $-NH-C(=NH)-N(C_2H_5)_2$, $-NH-C(=NH)-N(C_3H_7)_2$, -NH-C(=NH)-N(cyclo-$C_3H_5)_2$, $-NH-C(=NH)-N[CH(CH_3)_2]_2$, $-NH-C(=NH)-N[C(CH_3)_3]_2$, $-O-CO-NH_2$, $-O-CO-NHCH_3$, $-O-CO-NHC_2H_5$, $-O-CO-NHC_3H_7$, -O-CO-NH-cyclo-$C_3H_5$, $-O-CO-NH[CH(CH_3)_2]$, $-O-CO-NH[C(CH_3)_3]$, $-O-CO-N(CH_3)_2$, $-O-CO-N(C_2H_5)_2$, $-O-CO-N(C_3H_7)_2$, -O-CO-N(cyclo-$C_3H_5)_2$, $-O-CO-N[CH(CH_3)_2]_2$, $-O-CO-N[C(CH_3)_3]_2$, $-O-CO-OCH_3$, $-O-CO-OC_2H_5$, $-O-CO-OC_3H_7$, -O-CO-O-cyclo-$C_3H_5$, $-O-CO-OCH(CH_3)_2$, $-O-CO-OC(CH_3)_3$;

$R^{14}$ jusqu'à $R^{30}$ représentent indépendamment l'un de l'autre les résidus suivants:

$-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_3H_5$, cyclo-$C_4H_7$, cyclo-$C_5H_9$, cyclo-$C_6H_{11}$, cyclo-$C_7H_{13}$, cydo-$C_8H_{15}$, -Ph, $-CH_2$-Ph, $-CPh_3$, -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C_3H_6-CH=CH_2$, $-C_2H_4-CH=CH-CH_3$, $-CH_2-CH=CH-C_2H_5$, $-CH=CH-C_3H_7$,- $CH_2-CH=CH-CH=CH_2$, $-CH=CH-CH=CH-CH_3$, $-CH=CH-CH_2-CH=CH_2$, $-C(CH_3)=CH-CH=CH_2$, $-CH=C(CH_3)-CH=CH_2$, $-CH=CH-C(CH_3)=CH_2$, $-C_2H_4-C(CH_3)=CH_2$, $-CH_2-CH(CH_3)-CH=CH_2$, $-CH(CH_3)-CH_2-CH=CH_2$, $-CH_2-CH=C(CH_3)_2$, $-CH_2-C(CH_3)=CH-CH_3$, $-CH(CH_3)-CH=CH-CH_3$, $-CH=CH-CH(CH_3)_2$, $-CH=C(CH_3)-C_2H_5$, $-C(CH_3)=CH-C_2H_5$, $-C(CH_3)=C(CH_3)_2$, $-C(CH_3)_2-CH=CH_2$, $-CH(CH_3)-C(CH_3)=CH_2$, $-C(CH_3)=CH-CH=CH_2$, $-CH=C(CH_3)-CH=CH_2$, $-CH=CH-C(CH_3)=CH_2$, $-C_4H_8-CH=CH_2$, $-C_3H_6-CH=CH-CH_3$, $-C_2H_4-CH=CH-C_2H_5$, $-CH_2-CH=CH-C_3H_7$, $-CH=CH-C_4H_9$, $-C_3H_6-C(CH_3)=CH_2$, $-C_2H_4-CH(CH_3)-CH=CH_2$, $-CH_2-CH(CH_3)-CH_2-CH=CH_2$, $-CH(CH_3)-C_2H_4-CH=CH_2$, $-C_2H_4-CH=C(CH_3)_2$, $-C_2H_4-C(CH_3)=CH-CH_3$, $-CH_2-CH(CH_3)-CH=CH-CH_3$, $-CH(CH_3)-CH_2-CH=CH-CH_3$, $-CH_2-CH=CH-CH(CH_3)_2$, $-CH_2-CH=C(CH_3)-C_2H_5$, $-CH_2-C(CH_3)=CH-C_2H_5$, $-CH(CH_3)-CH=CH-C_2H_5$, $-CH=CH-CH_2-CH(CH_3)_2$, $-CH=CH-CH(CH_3)C_2H_5$, $-CH=C(CH_3)-C_3H_7$, $-C(CH_3)=CH-C_3H_7$, $-CH_2-CH(CH_3)-C(CH_3)=CH_2$, $-CH(CH_3)-CH_2-C(CH_3)=CH_2$, $-CH(CH_3)-CH(CH_3)-CH=CH_2$, $-CH_2-C(CH_3)_2-CH=CH_2$, $-C(CH_3)_2-CH_2-CH=CH_2$, $-CH_2-C(CH_3)=C(CH_3)_2$, $-CH(CH_3)-CH=C(CH_3)_2$, $-C(CH_3)_2-CH=CH-CH_3$, -CH(CH$_3$}-C(CH$_3$)=CH-CH$_3$, $-CH=C(CH_3)-CH(CH_3)_2$, $-C(CH_3)=CH-CH(CH_3)_2$, $-C(CH_3)=C(CH_3)-C_2H_5$, $-CH=CH-C(CH_3)_3$, $-C(CH_3)_2-C(CH_3)=CH_2$, $-CH(C_2H_5)-C(CH_3)=CH_2$, $-C(CH_3)$

$(C_2H_5)\text{-}CH{=}CH_2$, $\text{-}CH(CH_3)\text{-}C(C_2H_5){=}CH_2$, $\text{-}CH_2\text{-}C(C_3H_7){=}CH_2$, $\text{-}CH_2\text{-}C(C_2H_5){=}CH\text{-}CH_3$, $\text{-}CH(C_2H_5)\text{-}CH{=}CH\text{-}CH_3$, $\text{-}C(C_4H_9){=}CH_2$, $\text{-}C(C_3H_7){=}CH\text{-}CH_3$, $\text{-}C(C_2H_5){=}CH\text{-}C_2H_5$, $\text{-}C(C_2H_5){=}C(CH_3)_2$, $\text{-}C[C(CH_3)_3]{=}CH_2$, $\text{-}C[CH(CH_3)(C_2H_5)]{=}CH_2$, $\text{-}C[CH_2\text{-}CH(CH_3)_2]{=}CH_2$, $\text{-}C_2H_4\text{-}CH{=}CH\text{-}CH{=}CH_2$, $\text{-}CH_2\text{-}CH{=}CH\text{-}CH_2\text{-}CH{=}CH_2$, $\text{-}CH{=}CH\text{-}C_2H_4\text{-}CH{=}CH_2$, $\text{-}CH_2\text{-}CH{=}CH\text{-}CH{=}CH\text{-}CH_3$, $\text{-}CH{=}CH\text{-}CH_2\text{-}CH{=}CH\text{-}CH_3$, $\text{-}CH{=}CH\text{-}CH{=}CH\text{-}C_2H_5$, $\text{-}CH_2\text{-}CH{=}CH\text{-}C(CH_3){=}CH_2$, $\text{-}CH_2\text{-}CH{=}C(CH_3)\text{-}CH{=}CH_2$, $\text{-}CH_2\text{-}C(CH_3){=}CH\text{-}CH{=}CH_2$, $\text{-}CH(CH_3)\text{-}CH{=}CH\text{-}CH{=}CH_2$, $\text{-}CH{=}CH\text{-}CH_2\text{-}C(CH_3){=}CH_2$, $\text{-}CH{=}CH\text{-}CH(CH_3)\text{-}CH{=}CH_2$, $\text{-}CH{=}C(CH_3)\text{-}CH_2\text{-}CH{=}CH_2$, $\text{-}C(CH_3){=}CH\text{-}CH_2\text{-}CH{=}CH_2$, $\text{-}CH{=}CH\text{-}CH{=}C(CH_3)_2$, $\text{-}CH{=}CH\text{-}C(CH_3){=}CH\text{-}CH_3$, $\text{-}CH{=}C(CH_3)\text{-}CH{=}CH\text{-}CH_3$, $\text{-}C(CH_3){=}CH\text{-}CH{=}CH\text{-}CH_3$, $\text{-}CH{=}C(CH_3)\text{-}C(CH_3){=}CH_2$, $\text{-}C(CH_3){=}CH\text{-}C(CH_3){=}CH_2$, $\text{-}C(CH_3){=}C(CH_3)\text{-}CH{=}CH_2$, $\text{-}CH{=}CH\text{-}CH{=}CH\text{-}CH{=}CH_2$, $\text{-}C\equiv CH$, $\text{-}C\equiv C\text{-}CH_3$, $\text{-}CH_2\text{-}C\equiv CH$, $\text{-}C_2H_4\text{-}C\equiv CH$, $\text{-}CH_2\text{-}C\equiv C\text{-}CH_3$, $\text{-}C\equiv C\text{-}C_2H_5$, $\text{-}C_3H_6\text{-}C\equiv CH$, $\text{-}C_2H_4\text{-}C\equiv C\text{-}CH_3$, $\text{-}CH_2\text{-}C\equiv C\text{-}C_2H_5$, $\text{-}C\equiv C\text{-}C_3H_7$, $\text{-}CH(CH_3)\text{-}C\equiv CH$, $\text{-}CH_2\text{-}CH(CH_3)\text{-}C\equiv CH$, $\text{-}CH(CH_3)\text{-}CH_2\text{-}C\equiv CH$, $\text{-}CH(CH_3)\text{-}C\equiv C\text{-}CH_3$, $\text{-}C_4H_8\text{-}C\equiv CH$, $\text{-}C_3H_6\text{-}C\equiv C\text{-}CH_3$, $\text{-}C_2H_4\text{-}C\equiv C\text{-}C_2H_5$,- $CH_2\text{-}C\equiv C\text{-}C_3H_7$, $\text{-}C\equiv C\text{-}C_4H_9$, $\text{-}C_2H_4\text{-}CH(CH_3)\text{-}C\equiv CH$, $\text{-}CH_2\text{-}CH(CH_3)\text{-}CH_2\text{-}C\equiv CH$, $\text{-}CH(CH_3)\text{-}C_2H_4\text{-}C\equiv CH$, $\text{-}CH_2\text{-}CH(CH_3)\text{-}C\equiv C\text{-}CH_3$, $\text{-}CH(CH_3)\text{-}CH_2\text{-}C\equiv C\text{-}CH_3$, $\text{-}CH(CH_3)\text{-}C\equiv C\text{-}C_2H_5$, $\text{-}CH_2\text{-}C\equiv C\text{-}CH(CH_3)_2$, $\text{-}C\equiv C\text{-}CH(CH_3)\text{-}C_2H_5$, $\text{-}C\equiv C\text{-}CH_2\text{-}CH(CH_3)_2$, $\text{-}C\equiv C\text{-}C(CH_3)_3$, $\text{-}CH(C_2H_5)\text{-}C\equiv C\text{-}CH_3$, $\text{-}C(CH_3)_2\text{-}C\equiv C\text{-}CH_3$, $\text{-}CH(C_2H_5)\text{-}CH_2\text{-}C\equiv CH$, $\text{-}CH_2\text{-}CH(C_2H_5)\text{-}C\equiv CH$, $\text{-}C(CH_3)_2\text{-}CH_2\text{-}C\equiv CH$, $\text{-}CH_2\text{-}C(CH_3)_2\text{-}C\equiv CH$, $\text{-}CH(CH_3)\text{-}CH(CH_3)\text{-}C\equiv CH$, $\text{-}CH(C_3H_7)\text{-}C\equiv CH$, $\text{-}C(CH_3)(C_2H_5)\text{-}C\equiv CH$, $\text{-}C\equiv C\text{-}C\equiv CH$, $\text{-}CH_2\text{-}C\equiv C\text{-}C\equiv CH$, $\text{-}C\equiv C\text{-}C\equiv C\text{-}CH_3$, $\text{-}CH(C\equiv CH)_2$, $\text{-}C_2H_4\text{-}C\equiv C\text{-}C\equiv CH$, $\text{-}CH_2\text{-}C\equiv C\text{-}CH_2\text{-}C\equiv CH$, $\text{-}C\equiv C\text{-}C_2H_4\text{-}C\equiv CH$, $\text{-}CH_2\text{-}C\equiv C\text{-}C\equiv C\text{-}CH_3$, $\text{-}C\equiv C\text{-}CH_2\text{-}C\equiv C\text{-}CH_3$, $\text{-}C\equiv C\text{-}C\equiv C\text{-}C_2H_5$, $\text{-}C\equiv C\text{-}CH(CH_3)\text{-}C\equiv CH$, $\text{-}CH(CH_3)\text{-}C\equiv C\text{-}C\equiv CH$, $\text{-}CH(C\equiv CH)\text{-}CH_2\text{-}C\equiv CH$, $\text{-}C(C\equiv CH)_2\text{-}CH_3$, $\text{-}CH_2\text{-}CH(C\equiv CH)_2$, $\text{-}CH(C\equiv CH)\text{-}C\equiv C\text{-}CH_3$;

m est un numéro entier de 1 à 10;
n est un numéro entier de 0 à 5;
p est un numéro entier de 0 à 3;
q est un numéro entier de 0 à 4;
r représente 0 ou 1;
s représente 0 ou 1;
t est un numéro entier de 1 à 10.

**2.** Le ballonnet à cathéter selon la revendication 1, dans lequel l'au moins un agent anti-sténotique est intégré ou stocké dans l'au moins un agent dispersant moléculaire pour promouvoir le transport.

**3.** Le ballonnet à cathéter selon la revendication 1 ou 2, dans lequel le ratio entre l'agent anti-sténotique et l'au moins un agent dispersant moléculaire pour promouvoir le transport est de 90% en poids d'agent anti-sténotique à 10% en poids d'au moins un agent dispersant pour promouvoir le transport à 10% en poids d'agent anti-sténotique à 90% en poids d'au moins un agent dispersant pour promouvoir le transport.

**4.** Le ballonnet à cathéter selon l'une des revendications 1 - 3, dans lequel l'au moins un agent anti-sténotique est choisi parmi le groupe comprenant ou consistant en: paclitaxel, docetaxel, sirolimus, biolimus A9, zotarolimus, everolimus, myolimus, novolimus, pimecrolimus, tacrolimus, ridaforolimus, et temsirolimus.

**5.** Le ballonnet à cathéter selon la revendication 4, dans lequel l'agent anti-sténotique est paclitaxel.

**6.** Le ballonnet à cathéter selon la revendication 4, dans lequel l'agent anti-sténotique est sirolimus.

**7.** Le ballonnet à cathéter selon l'une des revendications 1 - 6, dans lequel le composé de formule générale (I) a un point d'ébullition d'au moins 500°C (à pression atmosphérique)

**8.** Le ballonnet à cathéter selon l'une des revendications 1 - 7, dans lequel le composé de formule générale (I) contient de 7 à 9 atomes d'oxygène.

**9.** Le ballonnet à cathéter selon l'une des revendications 1 - 8, dans lequel le composé de formule générale (I) a une masse molaire (poids moléculaire) d'au moins 500 g/mole.

**10.** Le ballonnet à cathéter selon l'une des revendications 1 - 9, dans lequel le composé de formule générale (I) a un point de fusion supérieur à -60°C.

**11.** Le ballonnet à cathéter selon l'une des revendications 1 - 10, dans lequel le composé de formule générale (I) a une

densité de 0,95 g/cm$^3$ à 1,05 g/cm$^3$.

**12.** Le ballonnet à cathéter selon l'une des revendications 1 - 11, dans lequel le composé de formule générale (I) a un point d'éclair supérieur à 100°C.

**13.** Le ballonnet à cathéter selon l'une des revendications 1 - 12, dans lequel le composé de formule générale (I) a un indice de réfraction $n_D^{20}$ compris entre 1,440 et 1,460.

**14.** Un procédé de fabrication d'un ballonnet à cathéter selon l'une des revendications 1 - 13, comprenant les étapes suivantes:

a) mise à disposition d'un ballonnet à cathéter d'un cathéter à ballonnet;
b) mise à disposition d'une solution de revêtement d'au moins un agent anti-sténotique et d'au moins un agent dispersant moléculaire pour promouvoir le transport dans un solvant ou dans un mélange de solvants ;
c) revêtement du ballonnet à cathéter avec la solution de revêtement par immersion, étalement, pulvérisation, revêtement aux pinceaux ou pipetage ;
d) séchage du revêtement appliqué.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2010055294 A1
- EP 2092942 A1
- WO 2004028582 A1
- EP 1189553 B1
- EP 0519063 B1
- WO 03059430 A1
- WO 9423787 A1
- EP 0383429 A
- WO 02043796 A2
- WO 03026718 A1
- WO 2008086794 A


### In der Beschreibung aufgeführte Nicht-Patentliteratur


- *CardioNews Letter,* 21. April 2006